# EUROPEAN PATENT SPECIFICATION

(11) **EP 2 685 943 B1**
(45) Date of publication and mention of the grant of the patent: **09.03.2016**
(21) Application number: 12711728.1
(22) Date of filing: 14.03.2012
(51) Int. Cl.: A61F 5/00, A61B 5/00

(54) **SELF-ADJUSTING GASTRIC BAND**
SELBSTREGELNDES MAGENBAND
ANNEAU GASTRIQUE À AUTO-AJUSTEMENT

(30) Priority: 16.03.2011 US 201113049453; 14.04.2011 WO PCT/US2011/032404; 31.05.2011 US 201113149585; 23.08.2011 US 201113216132
(43) Date of publication of application: 22.01.2014
(73) Proprietor: APOLLO ENDOSURGERY, INC., Austin, TX 78746 (US)
(72) Inventor: FRANKLIN, Ethan, Goleta, California 93117 (US); SNOW, Sean, Carpinteria, California 93013 (US); TORJESEN, Erik, Goleta, California 93117 (US); SCHWAB, Justin J., Santa Barbara, California 93105 (US); DOMINGUEZ, Zachary P., Santa Barbara, California 93101 (US)
(74) Representative: Hoffmann Eitle
(86) International application number: PCT/US2012/029025
(87) International publication number: WO 2012/125694

(56) References cited:
- WO-A2-2007/081304
- WO-A2-2008/058028
- US-A1- 2006 229 696

## Description

### FIELD

The present invention generally relates to medical systems for treating obesity and/or obesity-related diseases. More specifically, the present invention relates to gastric banding systems that may self-adjust to changes in a patient.

### BACKGROUND

Adjustable gastric banding apparatus have provided an effective and substantially less invasive alternative to gastric bypass surgery and other conventional surgical weight loss procedures. Despite the positive outcomes of invasive weight loss procedures, such as gastric bypass surgery, it has been recognized that sustained weight loss can be achieved through a laparoscopically-placed gastric band, for example, the LAP-BAND® (Allergan, Inc., Irvine, CA) gastric band or the LAP-BAND AP® (Allergan, Inc., Irvine, CA) gastric band. Generally, gastric bands are placed about the cardia, or upper portion, of a patient's stomach forming a stoma that restricts food's passage into a lower portion of the stomach. When the stoma is of an appropriate size that is restricted by a gastric band, food held in the upper portion of the stomach may provide a feeling of satiety or fullness that discourages overeating. Unlike gastric bypass procedures, gastric band apparatus are reversible and require no permanent modification to the gastrointestinal tract. An example of a gastric banding system is disclosed in Roslin, et al., U.S. Patent Pub. No. 2006/0235448.

Over time, a stoma created by a gastric band may need adjustment in order to maintain an appropriate size, which is neither too restrictive nor too passive. Accordingly, prior art gastric band systems provide a subcutaneous fluid access port connected to an expandable or inflatable portion of the gastric band. By adding fluid to or removing fluid from the inflatable portion by means of a hypodermic needle inserted into the access port, the effective size of the gastric band can be adjusted to provide a tighter or looser constriction.

However, the level of tightness of the gastric band may effect the patient's sensations and satisfaction level. In other words, if the gastric band is underfilled, the patient may experience hunger; but if the gastric band is overfilled (and thus "too tight"), the patient may experience tightness in the chest region, suffer from a food bolus blockage, and the like.

Sometimes, adjustment of a gastric band may be desirable in between adjustments made by a physician. For example, during normal operation of the gastric band, the band applies pressure to the outer surface of the upper stomach. However, it may be difficult to achieve the most effective level of tightness, and further, physicians may tend to err on the side of underfilling the gastric band (thereby decreasing efficacy of the gastric band system) as they consider the risk of overfilling the gastric band.

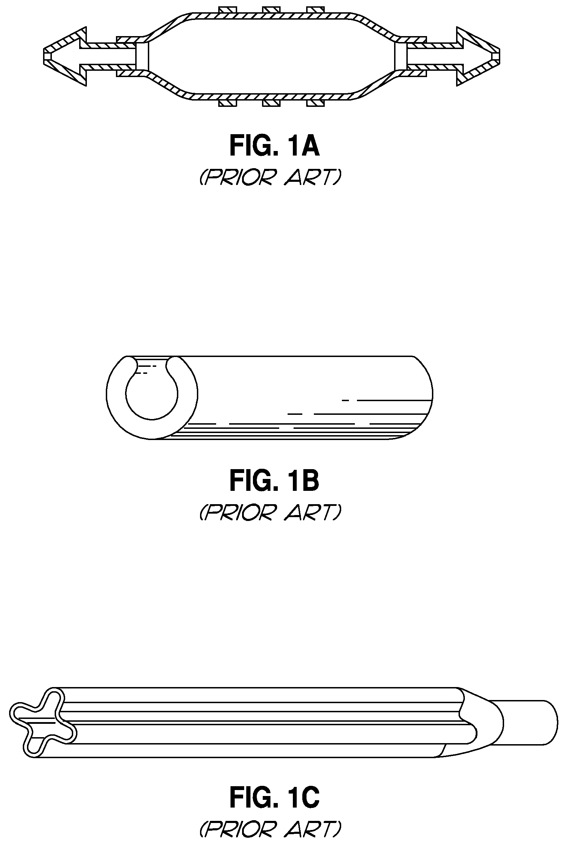

Some attempts have been made to develop a gastric band that promotes an effective fill level. For example, with reference to FIGS. 1A-1B, Lau, et al., U.S. Patent Pub. No. 2010/0191271 discloses an elastic bladder that is in constant fluid communication with the expandable balloon portion of a gastric band in order to continuously adjust the gastric band. With reference to FIG. 1C, Lau, et al., U.S. Patent Pub. No. 2010/0191265 discloses an alternative elastic bladder having four wings.

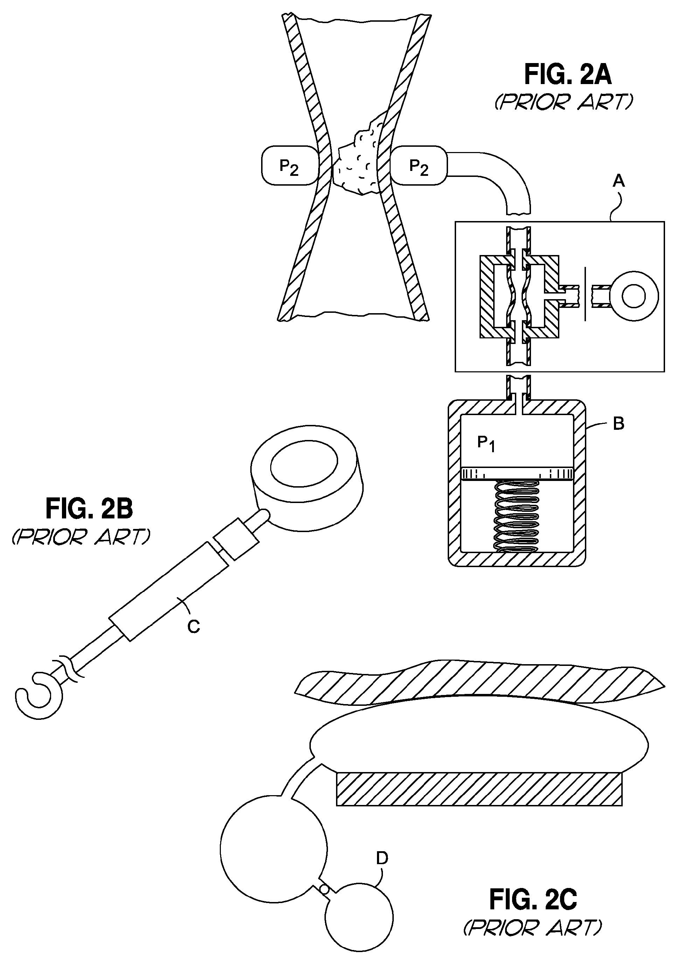

With reference to FIG. 2A, Coe, et al., U.S. Patent Pub. No. 2009/0216255 discloses a flow control device **A** that moves fluid between a hydraulic restriction system and a fluid source **B.** The additional flow control device **A** controls a rate of fluid flow between the restriction device and the fluid source **B.** With reference to FIG. 2B, Coe, et al., European Patent Application No. 2 074 970 A1 discloses a separate restriction device and pressure adjustment device **C.** The pressure adjustment device **C** regulates a constant force applied by the restriction device using, for example, a bellows and a spring.

With reference to FIG. 2C, Lechner, U.S. Patent Pub. No. 2009/0054914 discloses a controllable stomach band that has a chamber for controlling restriction of the stomach band. The chamber is coupled to a separate pressure chamber **D** that receives fluid leaving the chamber in the stomach band. The pressure chamber **D** is separated from the esophageal-gastric junction of the patient's stomach.

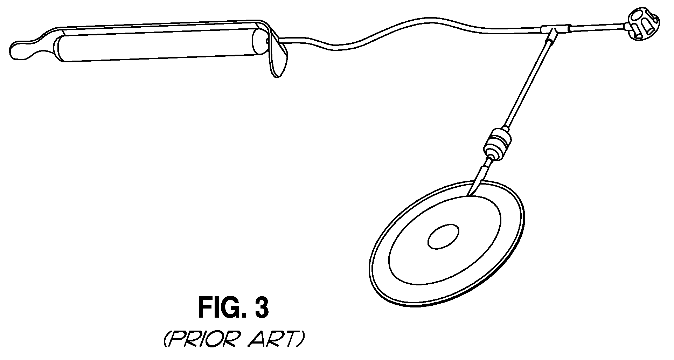

Further, with respect to FIG. 3, Steffen, U.S. Patent Pub. No. 2009/0062826 discloses an adjustable gastric band with a "conveyance device" that is powered by a "power storage device." The power storage device operates the conveyance device to move fluid between expandable chambers to adjust the gastric band.

Accordingly, it is desirable to develop a self-adjusting gastric band that will provide the needed pressure to the stomach to create the stoma and facilitate weight control, but that will also adapt and open up to allow a large bolus to pass through. It is further desirable to create an automatically self-adjusting gastric band that does not require an electrical power source and/or external adjustments, to allow a large bolus to pass through.

Additionally, it is desirable to make the adjustments without additional, complicated fluid control mechanisms, flow rate limiting devices, and/or valves to regulate the transfer of fluid within the self-adjusting gastric band. Moreover, it is desirable to make these adjustments to the gastric band utilizing compliant components to both reduce and restore the constriction of the gastric band.

WO 2007/081304 A2 discloses a self-regulating gastric band apparatus for adjusting stoma size in a patient. The apparatus includes an adjustable gastric band that has an expandable inner ring with a lumen or cavity for receiving a fluid. A band adjustment assembly is provided for implanting with the gastric band that includes a sensor for sensing a property of the gastric band, such as of the expandable inner ring. The band adjustment assembly further includes a pump assembly connected to the lumen of the expandable inner ring and to a controller that can operate the pump assembly to adjust the volume of the fluid in the lumen based on the sensed gastric band property. The band adjustment assembly includes memory storing an operating range for the particular band property, and the pump assembly is operated to maintain the sensed band parameter within the operating range.

### SUMMARY

This Summary is included to introduce, in an abbreviated form, various topics to be elaborated upon below in the Detailed Description.

Generally described herein are automatic, self-adjusting, gastric banding systems and improvements thereof that are capable of automatically relaxing and contracting in response to a large bolus passing through the area of a patient's stomach constricted by a gastric band. Alternatively, and/or in addition in one or more embodiments, the gastric banding systems described herein may also help prevent pouch dilatation and/or erosion. The apparatus and systems described herein may aid in facilitating obesity control and/or treating obesity-related diseases while generally being non-invasive once implanted. The automatic adjustments may also be made in response to other changes in the patient's esophageal-gastric junction, for example, in response to size, shape, and or location changes.

In one embodiment, a self-adjusting gastric band system for the treatment of obesity that adjusts to allow a bolus to pass through a constriction in a patient's stomach is disclosed. A bolus is, for example, any mass or object which may obstruct or at least partially obstruct any part of the gastrointestinal tract such as the lower esophagus, upper stomach or esophageal-gastric junction. The self-adjusting gastric band system comprises a gastric band having an inner portion, an outer portion, and an inflatable portion. The inner portion is configured to be placed around a portion of the patient's stomach to thereby create the constriction in the patient's stomach. The self-adjusting gastric band system also comprises a ring attached to the outer portion of the gastric band, the ring having a plurality of segmented portions, wherein each segmented portion includes an aperture, an access port fluidly coupled to the inflatable portion of the gastric band to fill and drain a fluid into or out of the inflatable portion, and a compliant reservoir fluidly coupled to the inflatable portion and the access port, the compliant reservoir capable of relaxing the constriction formed in the stomach by the gastric band by receiving fluid from the inflatable portion thereby allowing the bolus to pass through the relaxed constriction.

Further, the gastric band comprises a first compliant portion coupled to a part of the system. For example, the first compliant portion may be coupled to the inflatable portion, the access port, and/or the tubing. The first compliant portion automatically relaxes the constriction formed by the self-adjusting gastric band and allows the large bolus to pass through the constriction. After the bolus passes through the constriction, the gastric band automatically returns to its previous state.

In accordance with various embodiments, the first compliant portion facilitates automatically relaxing the constriction formed by the self-adjusting gastric band without causing a fluid to exit the inflatable portion of the gastric band. For example, the self-adjusting gastric band may comprise a ring coupled to the inflatable portion of the gastric band. The ring provides structure and support to the inflatable portion, and the ring facilitates disposing the inflatable portion about the esophageal-gastric junction.

The ring may be a flexible ring with a diameter that expands when a predetermined pressure is generated in the inflatable portion. For example, the predetermined pressure may be generated in response to the large bolus passing through the esophageal-gastric junction. The flexible ring expands to automatically relax the constriction formed by the self-adjusting gastric band. In various embodiments, the ring has a durometer in the range of approximately 20 to approximately 70.

According to a further embodiment, the first compliant portion receives a first amount of fluid from the inflatable portion when the large bolus causes a pressure in the first compliant portion to exceed an expansion pressure. Receiving the first amount of fluid from the inflatable portion facilitates relaxing the constriction formed by the self-adjusting gastric band and allowing the large bolus to pass through the constriction.

In an embodiment, the first compliant portion is fluidly coupled to the inflatable portion. The first compliant portion facilitates removing the first amount of fluid from the inflatable portion when the large bolus passes through the constriction.

According to another embodiment, the self-adjusting gastric band further comprises a second compliant portion fluidly coupled to the access port. The second compliant portion automatically removes a second amount of fluid from the inflatable portion via the access port to facilitate relaxing the constriction formed by the inflatable portion.

The tubing of the gastric banding system may be compliant tubing that expands in response to a pressure in the tubing exceeding a tubing expansion pressure when the large bolus passes through the constriction formed by the self-adjusting gastric band. In this regard, a third amount of fluid is removed from the inflatable portion when the compliant tubing expands. The tubing may be perforated to facilitate receiving the fluid from the inflatable portion via the tubing.

Further, another embodiment of the self-adjusting gastric band comprises a third compliant portion fluidly coupled to the tubing for automatically receiving a third amount of fluid from the inflatable portion via the tubing when the large bolus enters the esophageal-gastric junction. Receiving the third amount of fluid from the inflatable portion facilitates relaxing the constriction formed by the gastric band and allowing the large bolus to pass through the constriction.

The compliant components, according to various embodiments, comprise a kink-resisting feature. Further, the compliant components may comprise a leak-resisting feature. These components may be an elastic polymer, a balloon, a rubber container, a silicone container, a collapsible container, a bellows, and combinations thereof.

In an embodiment, a vacuum device may be used in transferring fluid from a gastric band to a reservoir to assist the patient in facilitating the passage of a large bolus through a constriction of the gastric band.

In an embodiment, a gastric banding system may include a tube with a gap or cut located within a balloon or reservoir. The gap or cut may allow for fluid transfer between fluidly-coupled components to the balloon or reservoir. In this manner, fluid may flow to the reservoir from an inflatable portion of the gastric banding system to relieve the pressure induced by the large bolus.

In an embodiment, a gastric banding system may include a tube with slits or holes located within a balloon or reservoir. The slits or holes may allow for fluid transfer between fluidly-coupled components to the balloon or reservoir. In this manner, fluid may flow to the reservoir from an inflatable portion of the gastric banding system to relieve the pressure induced by the large bolus.

In an embodiment, a gastric banding system may include a tube-like reservoir configured to inflate and deflate based on a volume level within the reservoir. The tube-like reservoir may be compliant and may have a star-shaped outer circumference in a first state and a circular, uniform outer circumference in a second state. Alternatively, or in addition, the middle portions of the tube-like reservoir may expand in diameter as more fluid is added.

In an embodiment, a gastric banding system may include a flattened reservoir having a uniform configuration, an indented configuration or a u-shaped configuration for inflating and deflating based on a volume level within the reservoir. In addition to providing a unique, expandable shape profiled over a continuous length, improved performance may be achieved through reduction of the effects of the external forces on fluid within an adjacent, non-compliant component.

In an embodiment, a gastric banding system may include a tube-on-tube reservoir having an outer non-compliant tube intended to prevent kinking, bending, or any other fluid disruption to an inner compliant tube. The inner compliant tube may be separated from the outer non-compliant tube by a gap which allows the inner compliant tube to expand (to fill the gap).

In an embodiment, a gastric banding system may include a reservoir with a winged portion, a coiled portion or an enlarged portion configured to inflate and deflate based on a volume level within the reservoir. These reservoirs may be attached or coupled to an access port of a gastric banding system.

In an embodiment, a gastric banding system may include a reservoir with internal structures such as a spring, a cage or a ring. The internal structures may act to prevent kinking, bending of the compliant portion, or otherwise prevent fluid flow interruptions within the reservoir.

In an embodiment, a gastric banding system may include a reservoir with external structures such as a skeleton or a protective layer. The external structures may act to prevent kinking, bending of the compliant portion, or otherwise prevent fluid flow interruptions within the reservoir.

In an embodiment, a gastric banding system may include a reservoir having depressions, pleatings or longitudinal structures along an outer circumferential perimeter. The depressions, pleatings or longitudinal structures may allow for easier and more predictable deflation of the reservoir, e.g., during implantation or removal procedures.

In an embodiment, a gastric banding system may have one or more reservoirs oriented radially from the gastric band.

In an embodiment, a gastric banding system may include non-saline fill substances such as a gel, a pseudoplastic material, or a Bingham plastic. The non-saline fill substances may have different properties that allow for different pressure behaviors when an external pressure is applied to the gastric banding system (e.g., when a large or small bolus is swallowed by the patient).

In an embodiment, a gastric banding system may be self-contained and may be filled with one or more of a various number of different fill substances. The self-contained gastric banding system may include a ring, one or more cushions, and one or more hinges that flex when a pressure is exerted on the cushions (e.g., when a patient swallows a large or small bolus of food).

In an embodiment, a gastric banding system may include a hybrid gas-saline component. The gas component may be a balloon or other gas filled member coated with a gas-impermeable coating and may be designed to flow between other components of the gastric banding system based on the pressure exerted, e.g., by a large bolus passing through a constriction formed by the gastric band.

In an embodiment, a gastric banding system may include an access port having a movable surface, which may move in response to a pressure change within the gastric banding system. For example, the movable surface may be a complaint portion that moves to increase the volume of the fluid portion (and therefore increases compliance) when the pressure increases within the gastric banding system (e.g., in response to a large bolus moving through a constriction of the gastric band). Once the pressure is reduced (e.g., the large bolus passing through the constriction), the movable surface may return to its original position, thereby decreasing the volume of the fluid portion of the access port. The access port may further include a septum, a fluid-permeable membrane, o-rings and a gas spring portion filled with a gas. Alternatively or in addition, the gas spring portion may be replaced with a wave spring, a cantilever spring, a constant force spring, a coil spring, a leaf spring, a Belleville spring, a hybrid polymer coil-air spring and the like.

In an embodiment, a gastric banding system may include a flow-rate control device. The flow rate control device may improve comfort of a patient when a large bolus is passing through the constriction by increasing the rate that fluid flows out of the gastric band and into the reservoir. In this manner, the pressure increase may be significantly reduced. Once the bolus passes, the reservoir-side pressure may gradually decrease as the gastric band begins to inflate again with fluid, and the gastric banding system may approach the equilibrium pressure.

In an embodiment, a gastric banding system may include a gastric band without a locking portion, and with a more inflexible ring portion. More particularly, instead of having a flexibly-stiff ring locked in place at an open end (as traditionally utilized in a standard gastric band), the gastric banding system does not include a locking portion and replaces the flexibly-stiff ring with a more inflexible ring such as a snap ring, a split ring, retaining ring and the like.

In an embodiment, a gastric banding system may include a ring and corresponding inflatable portions having a wider portion. The wider portion may operate to stimulate and restrict the patient's esophageal-gastric junction when the patient is not eating or swallowing small boluses. When the patient swallows medium-sized boluses, the wider portion may channel the medium bolus through the standard portions. And when the patient swallows large boluses, the wider portion may function to relieve the stress on the patient's tissue and assist to prevent formations of pouch dilatations.

In an embodiment, a gastric banding system may include a gastric band without a ring portion. The gastric band without a ring portion may be more flexible (e.g., by having decreased ring stiffness) than a standard gastric band, thereby resulting in a gastric banding system having muted pressure or force spikes on the tissues (e.g., in the esophageal-gastric junction) in a patient when the patient consumes a large bolus of food.

In an embodiment, a gastric banding system may include a gastric band with a modified ring portion. The modified ring portion may be more flexible (e.g., by having decreased ring stiffness) than a standard gastric band, thereby resulting in gastric banding systems having muted pressure or force spikes on the tissues (e.g., in the esophageal-gastric junction) in a patient when the patient consumes a large bolus of food.

In an embodiment, the ring and/or the belt of a standard or compliant gastric band may be modified to result in a band with increased compliance. For example, the ring and/or the belt may be modified with respect to material and/or geometry to increase compliance.

In an embodiment, a self-adjusting gastric band may comprise an inflatable portion disposable about an esophageal-gastric junction of the patient, a ring attached to the inflatable portion, the ring having a plurality of segmented portions, wherein each segmented portion includes an aperture, an access port fluidly coupled to the inflatable portion to fill and drain the inflatable portion, and a compliant reservoir fluidly coupled to the inflatable portion and the access port, the compliant reservoir for automatically relaxing the constriction formed by the self-adjusting gastric band and allowing the bolus to pass through the constriction by receiving fluid from the inflatable portion.

In an embodiment, a self-adjusting gastric band for the treatment of obesity that adjusts to allow a bolus of food to pass through a constriction in a patient's stomach formed by the self-adjusting gastric band, the self-adjusting gastric band comprising an inflatable portion disposable about an esophageal-gastric junction of the patient, a ring attached to the inflatable portion having a belt attached to a first end of the ring and a buckle attached to the second end of the ring configured to receive and secure the belt, the ring further comprising a tapered band between the belt and the buckle, an access port fluidly coupled to the inflatable portion to fill and drain the inflatable portion, and a compliant reservoir fluidly coupled to the inflatable portion and the access port, the compliant reservoir for automatically relaxing the constriction formed by the self-adjusting gastric band and allowing the bolus to pass through the constriction by receiving fluid from the inflatable portion.

In an embodiment, a self-adjusting gastric band may comprise a compliant inner tubing having a length and a diameter configured to be filled with saline, an outer shell disposed about the length of the compliant inner tubing and having a diameter greater than the diameter of the compliant inner tubing, and a compressible material completely filling a space between an outside surface of the compliant inner tubing and an inner surface of the outer shell.

In an embodiment, a self-adjusting gastric band may comprise an inflatable portion disposable about an esophageal-gastric junction of the patient, and a ring attached to the inflatable portion along an outer surface of the inflatable portion, the ring including a damping mechanism configured to resist displacement when a load greater than a predetermined threshold is applied, and to not resist displacement when a load smaller than a predetermined threshold is applied.

In an embodiment, a self-adjusting gastric band may comprise an inflatable portion disposable about an esophageal-gastric junction of the patient, a ring attached to the inflatable portion, a stretch limiter configured to limit the expansion of the inflatable portion, an access port fluidly coupled to the inflatable portion to fill and drain the inflatable portion and a compliant reservoir fluidly coupled to the inflatable portion and the access port, the compliant reservoir for automatically relaxing the constriction formed by the self-adjusting gastric band and allowing the bolus to pass through the constriction by receiving fluid from the inflatable portion.

### BRIEF DESCRIPTION OF THE DRAWINGS

The features, obstacles, and advantages of the present invention will become more apparent from the detailed description set forth below when taken in conjunction with the drawings, wherein:
FIG. 1A illustrates a prior art system that includes a elastic bladder.
FIG. 1B illustrates a prior art system that includes an elastic bladder having a fold.
FIG. 1C illustrates a prior art system that includes an elastic bladder having four wings.
FIG. 2A illustrates a prior art system that includes a flow rate limiting device.
FIG. 2B illustrates a prior art system that includes a fluid control mechanism.
FIG. 2C illustrates a prior art system that includes a valve and a chamber separated from the esophageal-gastric junction.
FIG. 3 illustrates a prior art system that includes a gastric band with a "conveyance device" that is powered by a "power storage device."
FIG. 4 illustrates an exploded, perspective view of a self-adjusting gastric banding system.
FIG. 5 illustrates an exploded, perspective view of a self-adjusting gastric banding system having various compliant components.
FIG. 6 illustrates an exploded, perspective view of another self-adjusting gastric banding system having various compliant components.
FIG. 7A illustrates a chart showing pressure-volume curves for a standard gastric band and a self-adjusting gastric band.
FIG. 7B illustrates a chart showing pressure-time curves for a standard gastric band and a self-adjusting gastric band subject to a period of obstruction.
FIG. 8A illustrates a vacuum device configured to provide suction on a patient's skin proximal to the compliant reservoir.
FIG. 8B illustrates the vacuum device of FIG. 8A in an operational state.
FIG. 9 illustrates a cross-sectional view of a compliant tubing-reservoir system.
FIG. 10 illustrates a cross-sectional view of a compliant tubing-reservoir system having a gap or cut sealed within a reservoir.
FIG. 11 illustrates a cross-sectional view of a compliant tubing-reservoir system having slits or holes sealed within a reservoir.
FIG. 12 illustrates an exploded, perspective view of a compliant tubing-reservoir system.
FIG. 13A illustrates an exploded, perspective view of a compliant tubing-reservoir system having a tube-shaped reservoir.
FIG. 13B illustrates a cross-sectional view of a non-extruded area of the tube-shaped reservoir of FIG. 13A.
FIG. 13C illustrates a cross-sectional view of an as-extruded area of the tube-shaped reservoir of FIG. 13A.
FIG. 14A illustrates a perspective view of a tube-shaped reservoir in a non-expanded state.
FIG. 14B illustrates a perspective view of the tube-shaped reservoir of FIG. 14A in an as-expanded state.
FIG. 15 illustrates an exploded, perspective view of a compliant tubing-reservoir system having a tube-shaped reservoir.
FIG. 15A illustrates a cross-sectional view of the tube-shaped reservoir of FIG. 15.
FIG. 16 illustrates a cross-sectional view of another tube-shaped reservoir.
FIG. 17A illustrates a cross-sectional view of a tube-shaped reservoir.
FIG. 17B illustrates a cross-sectional view of the tube-shaped reservoir of FIG. 17A in a second state.
FIG. 17C illustrates a cross-sectional view of the tube-shaped reservoir of FIG. 17A in a third state.
FIG. 18A illustrates a close-up view of a compliant tube-on-tube reservoir system.
FIG. 18B illustrates a cross-sectional view of the compliant tube-on-tube reservoir system of FIG. 18A.
FIG. 18C illustrates a partial, exploded view of the compliant tube-on-tube reservoir system of FIG. 18A.
FIG. 18D illustrates a partial, exploded view of the compliant tube-on-tube reservoir system of FIG. 18A.
FIG. 18E illustrates a cross-sectional view of the compliant tube-on-tube reservoir system of FIG. 18D.
FIG. 18F illustrates a perspective view of a portion of the compliant tube-on-tube reservoir system of FIG. 18A.
FIG. 18G illustrates a partial, exploded view of the compliant tube-on-tube reservoir system of FIG. 18A.
FIG. 19A illustrates a perspective view of a coiled reservoir in a first state.
FIG. 19B illustrates a perspective view of the coiled reservoir of FIG. 19A in a second state.
FIG. 20A illustrates a perspective view of a reservoir having winged portions in a first state.
FIG. 20B illustrates a perspective view of the reservoir having winged portions of FIG. 20A in a second state.
FIG. 21A illustrates a perspective view of a tube-shaped reservoir having a closed end in a first state
FIG. 21B illustrates a perspective view of the tube-shaped reservoir having a closed end of FIG. 21A in a second state.
FIG. 21C illustrates a perspective view of a tube-shaped reservoir having a closed end of FIG. 21A in a third state.
FIG. 22 illustrates a perspective view of a reservoir having an internal spring.
FIG. 23 illustrates a perspective view of a reservoir having an internal cage.
FIG. 24 illustrates a perspective view of a reservoir having internal rings.
FIG. 25 illustrates a perspective view of a reservoir having an external shell.
FIG. 25A illustrates a cross-sectional view of the reservoir having the external shell of FIG. 25.
FIG. 26 illustrates a cross-sectional view of another reservoir having an external shell.
FIG. 27 illustrates a perspective, cross-sectional view of a reservoir having depressions.
FIG. 28 illustrates a cross-sectional view of a reservoir having depressions
FIG. 29 illustrates a cross-sectional view of a reservoir having depressions
FIG. 30A illustrates a top view of a gastric banding system having a compliant reservoir about the circumference of the gastric band.
FIG. 30B illustrates a side view of the gastric banding system of FIG. 30A.
FIG. 31 illustrates a side view of a gastric banding system of having a compliant reservoir about the circumference of the gastric band.
FIG. 32 illustrates an orientation of a gastric banding system having a plurality of reservoirs extending radially from the gastric band.
FIG. 33A illustrates a large bolus/small bolus pressure-time graph for two distinct gastric banding systems.
FIG. 33B illustrates a large bolus/small bolus pressure-time graph for two distinct gastric banding systems.
FIG. 34A illustrates a perspective view of a self-contained gastric banding system.
FIG. 34B illustrates a top view of the self-contained gastric banding system of FIG. 34A.
FIG. 35 illustrates a top perspective view of another self-contained gastric banding system.
FIG. 36 illustrates an exploded, perspective view of a gastric banding system having gas-impermeable components.
FIG. 37A illustrates an exploded, perspective view of a hybrid gas-fluid gastric banding system having gas-impermeable components.
FIG. 37B illustrates an exploded, perspective view of a hybrid foam-fluid gastric banding system having gas-impermeable components.
FIG. 38A illustrates an exploded, perspective view of a hybrid gas-fluid gastric banding system having a gas-spring.
FIG. 38B illustrates a close-up view of the gas-spring within a reservoir of FIG. 38A.
FIG. 38C illustrates a close-up view of the gas-spring of FIG. 38A.
FIG. 38D illustrates a cross-sectional view of the gas-spring of FIG. 38A.
FIG. 39 illustrates a close up view of an access port for a gastric banding system.
FIG. 40 illustrates a close up view of another access port for a gastric banding system.
FIG. 41 illustrates a close up view of another access port for a gastric banding system.
FIG. 42 illustrates a perspective view of a gastric banding system having a flow rate restrictor.
FIG. 43 illustrates a time-pressure curve for a gastric banding system without a flow rate restrictor and a time-pressure curve for a gastric banding system with a flow rate restrictor.
FIG. 44A illustrates an always-open gastric banding system.
FIG. 44B illustrates an always-open gastric banding system having an elastic member.
FIG. 45 illustrates a cross-sectional view of a gastric band located about an esophageal-gastric junction of a patient.
FIG. 46 illustrates a cross-sectional view of a gastric band having a funnel shape.
FIG. 47A illustrates an exploded, perspective view of a gastric banding system including a gastric band without a ring.
FIG. 47B illustrates a perspective view of the gastric band of FIG. 47A.
FIG. 48A illustrates an exploded, perspective view of a gastric banding system including a gastric band having a ring with holes.
FIG. 48B illustrates a perspective view of the gastric band of FIG. 48A.
FIG. 49A illustrates an exploded, perspective view of a gastric banding system including a gastric band having a ring with cut-out portions.
FIG. 49B illustrates a perspective view of the gastric band of FIG. 49A.
FIG. 50A illustrates an exploded, perspective view of a gastric banding system including a gastric band having a modified ring according an embodiment of the present invention.
FIG. 50B illustrates a perspective view of the gastric band of FIG. 50A according to an embodiment of the present invention.
FIG. 51A illustrates an exploded, perspective view of a gastric banding system including a gastric band having a modified ring according an embodiment of the present invention.
FIG. 51B illustrates a perspective view of the gastric band of FIG. 51A according to an embodiment of the present invention.
FIG. 52A illustrates a close-up perspective view of a belt of a gastric band.
FIG. 52B illustrates a close-up perspective view of a belt of a gastric band.
FIG. 52C illustrates a close-up perspective view of a belt of a gastric band.
FIG. 52D illustrates a close-up perspective view of a belt of a gastric band.
FIG. 53A illustrates an exploded, perspective view of a gastric banding system including a gastric band without a ring.
FIG. 53B illustrates a perspective, cross-sectional view of the gastric band of FIG. 53A.
FIG. 54A illustrates a cross sectional view of a gastric band.
FIG. 54B illustrates a close-up, cross-sectional view of the gastric band of FIG. 54A.
FIG. 55 illustrates a gastric band featuring dual connected pistons.
FIG. 56 illustrates a gastric band featuring connected displacing chambers.
FIG. 57 illustrates a gastric band featuring interlaced leaves.
FIG. 58 illustrates a gastric band featuring a single damping piston.
FIG. 59A illustrates a gastric band featuring a force limit release device in a closed position.
FIG. 59B illustrates a gastric band featuring a force limit release device in an open position.
[FIG. 60 illustrates a gastric band featuring an encapsulated stretch limiter.
FIG. 61 illustrates a gastric band featuring an external stretch limiter.
FIG. 62 illustrates a gastric band featuring an internal stretch limiter.

### DETAILED DESCRIPTION

Apparatuses, systems and/or methods that implement the embodiments of the various features of the present invention will now be described with reference to the drawings. The drawings and the associated descriptions are provided to illustrate some embodiments of the present invention and not to limit the scope of the present invention. Throughout the drawings, reference numbers are re-used to indicate correspondence between referenced elements.

The present invention generally provides self-adjusting gastric banding systems, for example, for treatment of obesity and obesity related conditions, as well as systems for allowing automatic self-adjustment of gastric bands when a patient swallows a large bolus.

Self-adjusting gastric bands are effective in helping a patient lose weight when the band is properly tightened around the patient's esophageal-gastric junction. During normal operation, the band applies pressure to the outer surface of the upper stomach. But, in some instances, the patient may swallow a bolus which is too large to pass through the constriction produced by the band - for example, when the patient swallows a large piece of steak. The result can be a painful experience which, if it persists, may require medical intervention to release the blockage.

In accordance with various embodiments of the present invention, the self-adjusting gastric band provides the needed pressure to the stomach to encourage weight loss. However, when a large bolus of food is swallowed, the self-adjusting gastric band temporarily and automatically opens up to allow the bolus through. After the bolus passes through, the mechanisms within the band return the band to its original size and shape. In an embodiment, electrical power and/or power external to the patient is not utilized to perform these adjustments. Further, in an embodiment, complicated fluid control mechanisms, flow rate limiting devices, and/or valves are not utilized to regulate the transfer of fluid within the self-adjusting gastric band.

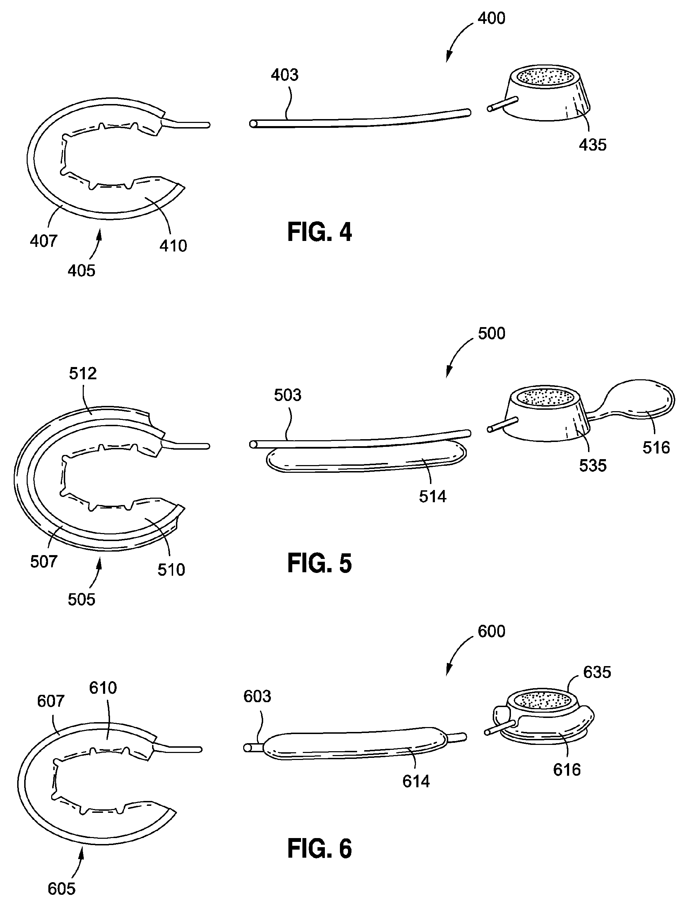

Turning now to FIG. 4, a self-adjusting gastric banding system **400** includes a gastric band **405** coupled to a subcutaneous injection port **435** via tubing **403.** The gastric band **405** includes a circular ring **407** and an inflatable portion **410** disposed on the inside of the ring **407.** The inflatable portion **410** separates the patient's stomach from the ring **407** when the gastric band **405** is implanted around the esophageal-gastric junction of the patient's stomach. The ring **407** provides structure and support to the inflatable portion **410,** and facilitates implanting the gastric band **405** around the patient's stomach.

The access port **435** may be sutured onto the rectus muscle sheath or any other conveniently accessible muscle. The rectus muscle sheath provides a secure surface on which to attach the access port **435** under a layer of fat that separates the patient's skin from the muscle.

The inflatable portion **410** may be filled and drained with a fluid via the tubing **403.** For example, the tubing **403** may be connected to the subcutaneous access port **435** for filling and draining the inflatable portion **410** via subcutaneous injections. The inflatable portion **410** may also be coupled to a reservoir to facilitate automatic adjustment of the inflatable portion **410,** and the constriction it causes, when a large bolus attempts to pass through the constriction. When more fluid is introduced in the inflatable portion **410,** the constriction around the stomach generally becomes tighter. Correspondingly, when less fluid is present, the constriction loosens and/or opens up.

The fluids used within the gastric band **405** may include any fluid that is biocompatible and incompressible. The fluid has no adverse effect on the patient in the unlikely event that a leak emanates from the system. The fluid can simply be water or any biocompatible polymer oil such as caster oil. In an example embodiment, the fluid is saline, a drug, and/or combinations thereof.

The ring **407** is designed to be a compliant portion of the gastric band **405.** For example, the ring **407** may flex and/or expand in response to a bolus of food moving through the constriction caused by the gastric band **405.** The ring **407** may have flexible components and rigid components, such that the flexible components expand when a certain elevated and/or maximum pressure is reached in the inflatable portion **410.** This elevated pressure may exist due to the presence of an obstruction such as a bolus near the gastric band **405.** As the ring **407** expands, the diameters of the ring **407** and the inflatable portion **410** increase, and the constriction on the stomach due to the gastric band **405** is reduced to allow the bolus to pass through. When the bolus has passed, the elevated pressure no longer exists, and the gastric band **405** returns to the pre-obstruction state.

The entire ring **407** may be flexible and/or expandable such that a diameter of the ring **407** increases in response to the elevated pressure in the inflatable portion **410.** For example, the ring **407** may be constructed of silicone that has a durometer in the range of approximately 20 to approximately 70.

It should be understood that the flexible ring **407** and the other mechanisms disclosed herein for automatically adjusting the constriction of the gastric band **405** are only example embodiments. Any mechanism for automatically adjusting the constriction of the gastric band **405** that does not include electrical power, power external to the patient, complicated fluid control mechanisms, flow rate limiting devices, and/or valves is contemplated within the scope of the present invention. As an example, the term "automatically" refers to situations when the compliant member expands, moves, contracts or is altered without the use of an electronic device causing the change.

With reference to FIG. 5, various compliant components may be utilized to automatically adjust the constriction of the gastric band **505** about the esophageal-gastric junction of the patient's stomach. Although three compliant components are illustrated in FIG. 5, as noted above, one or more of the components may be present in various systems.

For example, in a system, a band compliant component **512** is fluidly coupled to the inflatable portion **510** of the gastric band **505.** The compliant component **512** is located on the outside of the ring **507,** opposite the inflatable portion **510,** and may be coupled to the ring **507** and the inflatable portion **510.** Further, in a system, one or more fluid ports may extend from the inflatable portion **510** to the compliant component **512** to fluidly couple the inflatable portion **510** to the compliant component **512.**

With reference to FIGS. 5 and 6, a tube compliant component **514, 614** may be fluidly coupled to the tubing **503, 603.** As illustrated in FIG. 6, the compliant component **614** may run along substantially the entire length of the tubing **603.** As illustrated in FIG. 5, the compliant component **514** may be limited to a smaller section of the entire length of the tubing **503.** The compliant component **514, 614** may be fluidly coupled to the tubing **503** at one or more locations. For example, with reference to FIG. 6, the compliant component **614** and the tubing **603** may be perforated to allow for efficient transfer of the fluid between the tubing **603** and the compliant component **614.**

The tubing **603** itself may be compliant, and the durometer, thickness, and/or diameter of the tubing **603** may be altered to achieve a desired degree of compliance. Other components of the gastric band **605** may similarly have altered properties in order to achieve a desired degree of compliance.

In a system where the tube compliant component **514, 614** facilitates automated adjustment of the gastric band **505, 605,** the compliant component **514, 614** may have features configured to resist kinking and/or leakage of the tubing **503, 603.** For example, the compliant component **514, 614** may include rigid portions (*e.g*., similar to a skeleton) and flexible portions. The rigid components may give structure to the compliant component **514, 614** and/or the tubing **503, 603** to prevent kinking and/or leakage due to external forces on the compliant component **514, 614** and/or the tubing **503, 603.** The flexible components may automatically expand in response to an increased pressure in the inflatable portion **510, 610** of the gastric band **505, 605.**

With continued reference to FIGS. 5 and 6, the access port **535, 635** may be fluidly coupled to a port compliant component **516, 616.** As illustrated in FIG. 5, the compliant component **516** may be a balloon, reservoir, or other expandable device that is adjacent to the port **535.** As illustrated in FIG. 6, the compliant component **616** may substantially surround the access port **635.** The compliant component **616** may be fluidly coupled to the access port **635** at a single location near a coupling between the tubing **603** and the access port **635.** The compliant component **616** may be fluidly coupled to the access port **635** at multiple locations.

As noted above, any combination of the inflatable portion compliant component **512,** a compliant ring **407,** the tube compliant component **514, 614,** and/or the port compliant component **516, 616** may be used. When the pressure in the inflatable portion **510, 610** exceeds a predetermined pressure, the compliant components **407, 512, 514, 516, 614, 616,** in any particular configuration or combination, expand to receive an amount of the fluid from the inflatable portion **510, 610** via the inflatable portion **510, 610,** the tubing **503, 603,** and/or the access port **535, 635,** and/or to reduce the constriction formed by the gastric band **405, 505, 605.** The predetermined pressure may be predetermined based on a pressure that would indicate an obstruction is attempting to pass through the constriction caused by the gastric band **405, 505, 605.**

The compliant components **407, 512, 514, 516, 614, 616** described herein, may be designed with an expansion pressure at which pressure the components **407, 512, 514, 516, 614, 616** begin to expand, to receive fluid from the inflatable portion **510, 610** of the gastric band **505, 605,** and/or to reduce the constriction formed by the gastric band **405, 505, 605.** The expansion pressure may be configured to correspond to a predetermined pressure in the inflatable portion **410, 510, 610** that may indicate an obstruction exists in the esophageal-gastric junction.

For example, the obstruction may result in a large spike in intra-esophageal pressure that exceeds the expansion pressure and causes the compliant components to expand and receive fluid from the inflatable portion **510, 610.** The reduction in fluid in the inflatable portion **510, 610** causes the constriction around the patient's stomach to loosen, in order to relieve the spike in pressure and allow the obstruction to pass through the esophageal-gastric junction. When the obstruction passes, the increased pressure in the inflatable portion **510, 610** is reduced, and the fluid flows back into the inflatable portion **510, 610** due to the elasticity of the compliant components **512, 514, 516, 614, 616,** to restore the original amount of constriction of the gastric band **505, 605.** This change in constriction of the gastric band **505, 605** results or is achieved without the use of flow rate limiting devices or valves.

The various compliant components disclosed herein may have any shape or configuration that facilitates removing an amount of fluid from the inflatable portion of the gastric band in response to an increased pressure in the inflatable portion. For example, the compliant components may be selected from a group consisting of a compressible reservoir, an elastic polymer, a balloon, a rubber container, a silicone container, a collapsible container, a bellows, and combinations thereof that are configured to contain the fluid.

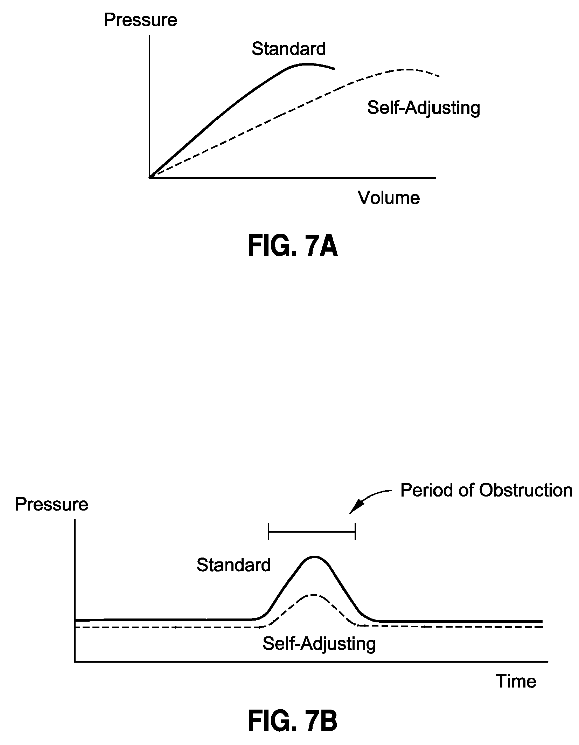

The graph in FIG. 7A illustrates the effect the compliant components described herein have on the pressure in the gastric banding system. As can be seen in FIG. 7A, a standard gastric banding system without compliant components has a certain pressure-volume relationship. After the gastric banding system is flushed with saline to remove any air trapped within the system (e.g., in the gastric band, the tubing, and the port), the pressure-volume relationship generally takes the form illustrated by the "Standard" curve in FIG. 7A. The dashed "Compliant" curve illustrates an example embodiment of the pressure-volume relationship for a gastric banding system with one or more compliant components. As illustrated, the self-adjusting gastric banding system may include a greater volume of saline than a standard gastric banding system for a given level of pressure.

The graph in FIG. 7B illustrates pressure characteristics of a "Standard" gastric banding system and a "Self-Adjusting" gastric banding system during use of the systems in a patient. Initially, the two systems are set to the same operating pressure, for example, for a desired level of constriction of the patient's stomach. As a large bolus of food or some other obstruction encounters the gastric band, the pressure in each system increases. As illustrated, the standard system has a larger pressure increase during the period of obstruction than the self-adjusting gastric banding system experiences. This smaller increase in pressure, according to various embodiments, is due to the addition of the reservoir space in the compliant component(s). As pressure in the gastric banding system increases, fluid is transferred into the reservoir space. Once the obstruction passes, the fluid is automatically returned from the reservoir space back into the gastric band.

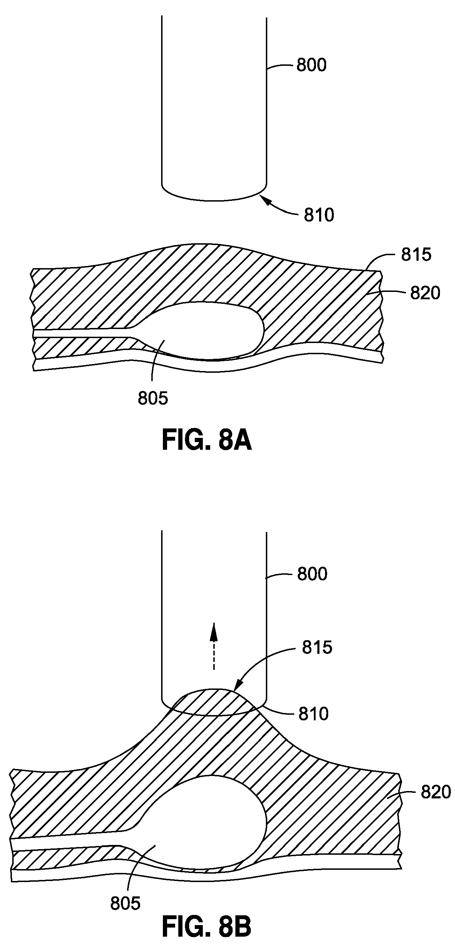

FIG. 8A illustrates a vacuum device **800** which may be used to assist the patient in transferring fluid into a reservoir **805** from a gastric band (not shown) to allow a large bolus to pass through the constriction of the gastric band. The vacuum device **800** may include a tip **810,** which in one embodiment, may have a diameter substantially equal to the diameter of the reservoir **805.**

As shown in FIG. 8B, when the vacuum device **800** is activated and brought close to a patient's skin **815,** the vacuum device **800,** by using suction, may form a seal on a contacted area of the patient's skin **815** and slightly pull or tug on the patient's skin **815** and a subcutaneous fat layer **820** located below the skin **815.** The vacuum device **800** may be configured to provide suction strength of differing magnitudes, but without harming the patient. Since there is no pathway for air or bodily fluids to collect between the subcutaneous fat layer **820** of the patient and the top surface of the reservoir **805,** the top surface of the reservoir **805** may be pulled towards the vacuum device **800** along with the subcutaneous fat layer **820.** As a result of the suction created, fluid may flow out of the gastric band (not shown) and into the reservoir **805** thereby relaxing a constriction caused by the gastric band and allowing a bolus of food to pass through the constriction.

Specific compliant self-adjusting gastric banding systems having been described, attention will now be turned to additional and/or alternative improvements which may be integrated and/or implemented with any number of obesity-preventing systems, including the self-adjusting gastric banding systems described herein.

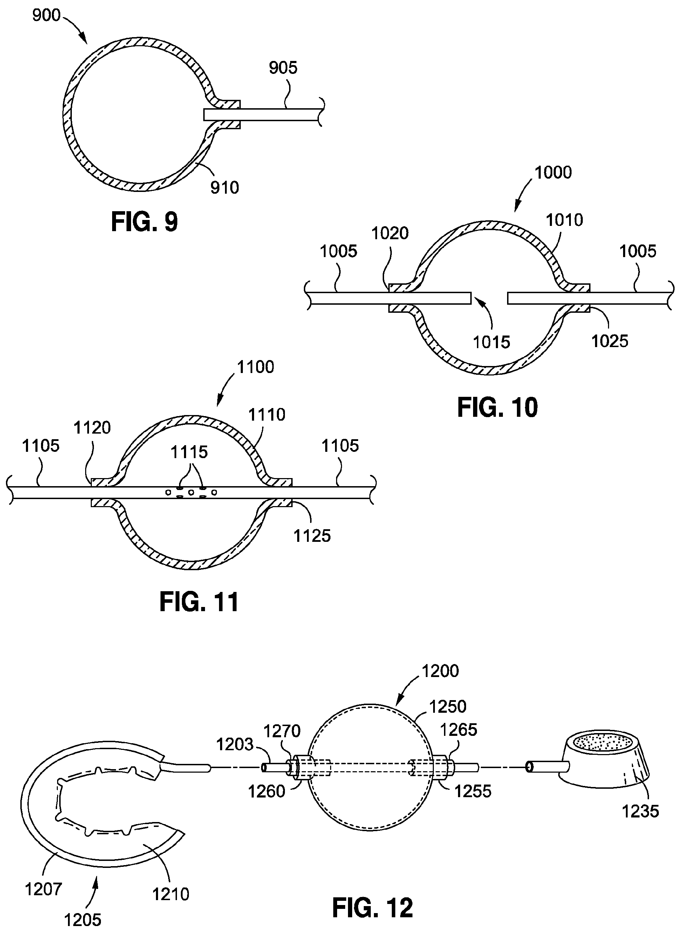

Turning to FIG. 9, a close-up view of a compliant tubing-reservoir system **900** is illustrated. The compliant tubing-reservoir system **900** may include a tubing **905** which terminates at or inside a balloon or a reservoir **910.** In one embodiment, the tubing (not shown in FIG. 5) may connect an injection port (e.g., injection port **535** of FIG. 5) and a reservoir (e.g., port compliant portion **516** of FIG. 5) thereby allowing a fluid path to travel through the tubing **905** to the reservoir **910.** While shown here in FIG. 9 to terminate in the reservoir **910,** the tubing **905** may, in one embodiment, continue and pass through. In operation, the tubing-reservoir system **900** may relieve increases in pressure, for example, generated by the passing of a large bolus swallowed by the patient. The relief may result from the transfer of fluid or other substances from an inflatable portion (not shown) through the tubing **905** and into the elastically deformable balloon or reservoir **910.**

FIG. 10 illustrates a fluid path connecting component for a pass-through tubing-reservoir system **1000** where a tubing **1005** passes through both a first opening **1020** and a second opening **1025** of a balloon or reservoir **1010.** Here, the tubing **1005** may include a gap or cut **1015** inside the reservoir **1010** (which creates a fluid path between the tubing **1005** and the reservoir **1010**). The gap **1015** is sealed within the reservoir **1010** such that the addition of fluid may enlarge the reservoir **1010** while the reduction of fluid may shrink or decrease the size of the reservoir **1010.** In operation, the tubing-reservoir system **1000** may relieve increases in pressure, for example, generated by the passing of a large bolus swallowed by the patient. The relief may result from the transfer of fluid or other substances from an inflatable portion (not shown) through the tubing **1005** and into the elastically deformable reservoir **1010.**

FIG. 11 illustrates a pass-through tubing-reservoir system **1100** where a tubing **1105** passes through both a first opening **1120** and a second opening **1125** of a balloon or reservoir **1110.** The tubing **1105** is shown with a series of holes or slits **1115** located at a position inside the reservoir **1110** which allows fluid and/or other substances to exit the tubing **1105** and into the reservoir **1110.** The same holes or slits **1115** further allow fluid and/or other substances to enter the tubing **1105** from the reservoir **1110,** thereby creating the fluid path between the tubing **1105** and the reservoir **1110.** In operation, the tubing-reservoir system **1100** may relieve increases in pressure, for example, generated by the passing of a large bolus swallowed by the patient. The relief may result from the transfer of fluid or other substances from an inflatable portion (not shown) through the tubing **1105** and into the elastically deformable balloon or reservoir **1110.**

FIG. 12 illustrates a compliant pass-through tubing-reservoir system **1200** where a tubing **1203** passes through both a first opening **1255** and a second opening **1260** of a balloon or reservoir **1250.** The system **1200** may also include a first collar **1265** and a second collar **1270** for facilitating the connection of the reservoir **1250** to the tubing **1203.** The tubing **1203** may be fixed to the reservoir **1250** by the customizably-fitted first collar **1265** and the customizably-fitted second collar **1270.** Here, the tubing **1203** and/or the reservoir **1250** may be compliant components creating a fluid path between a gastric band **1205** and a port **1235.** In one embodiment, the gastric band **1205** and the port **1235** may be the gastric band **405** and the port **435** of FIG. 4, respectively. The gastric band **1205** may include a circular ring **1207** and an inflatable portion **1210.** Similarly, the circular ring **1207** and the inflatable portion **1210** may be the circular ring **407** and the inflatable portion **410** of FIG. 4, respectively. In operation, the tubing-reservoir system **1200** may relieve increases in pressure, for example, generated by the passing of a large bolus swallowed by the patient. The relief may result from the transfer of fluid or other substances from the inflatable portion **1210** through the tubing **1203** and into the elastically deformable reservoir **1250.**

The balloons/reservoirs **910, 1010, 1110** and **1250** may be a spherical balloon constructed of biocompatible, elastomeric material. However, the balloon/reservoirs **910, 1010, 1110** and **1250** may be any other shape (e.g., a cylindrical balloon, etc.).

Each of the tubing-reservoir systems **900, 1000, 1100** and **1200** may be considered a compliant system having one or more compliant components. Moreover, the systems **900, 1000, 1100** and **1200** may be arranged with other components of a gastric banding system (e.g., gastric banding system **500** or **600**) in series or in parallel with each other to optimize the overall compliance, and to add redundancy to the gastric banding system (e.g., gastric banding system **500** or **600**), if desired.

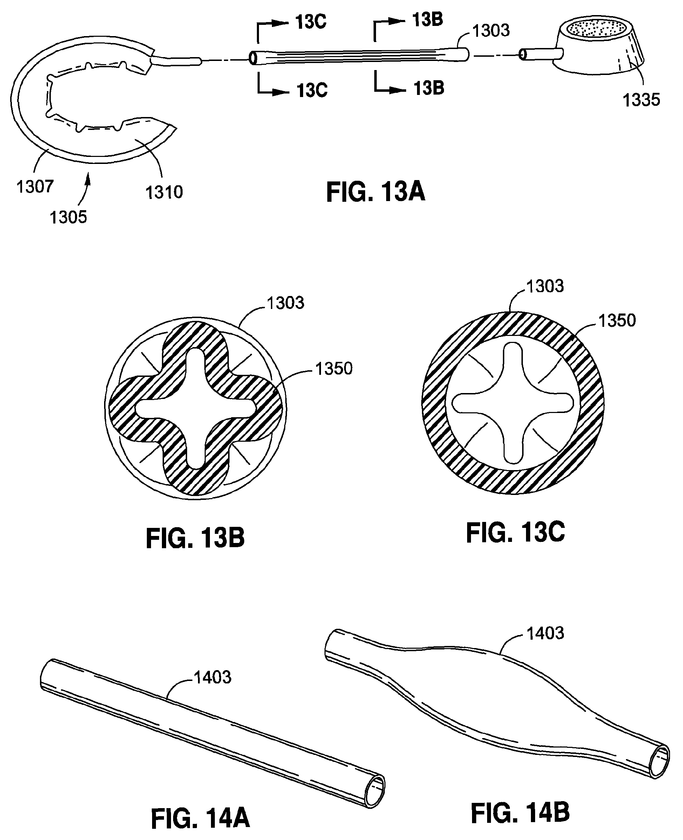

FIGS. 13A-C illustrate a compliant gastric banding system **1300** having a compliant reservoir **1303.** The compliant gastric banding system **1300** may further include a gastric band **1305** having a ring **1307** and an inflatable portion **1310** fluidly coupled to an access port **1335** via the reservoir **1303.** As shown, the reservoir **1303** may have a tube-like appearance and may replace a balloon-type reservoir (e.g., reservoir/balloon **910, 1010, 1110, 1250**) as a compliant component in any gastric banding system (e.g., gastric banding systems **900, 1000, 1100, 1200**). FIG. 13A generally illustrates the reservoir **1303** in an as-extruded view. The reservoir **1303,** as further shown by FIGS. 13B and 13C, may include a star-shaped structure defining an outer circumference **1350** and may be sized to expand when holding fluid that is displaced from an adjacent non-compliant fluid-carrying device (e.g., the inflatable portion **1310**). In operation, the reservoir **1303** may include star-shaped folds when in a natural, deflated state as shown in FIG. 13B. However, as fluid and/or other substances begin to move into the reservoir **1303** and exert pressure on the inner walls, the star-shaped folds may deform and expand, thereby changing the appearance of the outer circumference **1350** as shown in FIG. 13C. Here, the outer circumference **1350** may appear more similar to a normal, cylindrical tube. As the fluid moves out of the reservoir **1303,** the star-shaped folds may begin to reappear and the outer circumference **1350** may revert back to its star-shaped form as shown in FIG. 13B. The reservoir **1303** may be constructed from a polymer such as silicone, in various durometers so as to expand at a controlled rate.

FIGS. 14A and 14B illustrate a compliant tube-like reservoir **1403** in an as-molded state (e.g., when not overly filled with a fluid) and an expanded state (e.g., when filled with a fluid above a threshold volume), respectively. The reservoir **1403** may replace a balloon-type reservoir (e.g., reservoir/balloon **910, 1010, 1110, 1250**) or another tube-like reservoir (e.g., reservoir **1303**) as a compliant component for any gastric banding system (e.g., gastric banding systems **900, 1000, 1100, 1200, 1300**)**.** In operation, the reservoir **1403** may appear very similar to a non-compliant, stiff tube when in a natural, as-molded state as shown in FIG. 14A. However, as fluid and/or other substances begin to move into the reservoir **1403** and exert pressure on the inner walls, the elastic wall of the reservoir **1403** may begin to deform and expand, thereby appearing more similar to the expanded state as shown in FIG. 14B. As the fluid moves out of the reservoir **1403,** the elastic walls may begin to regain its original, as-molded appearance. Similar to the reservoir **1303** of FIGS. 13A-C, the reservoir **1403** may be constructed from a polymer such as silicone, in various durometers so as to expand at a controlled rate. In one embodiment, the reservoir **1403** may have a uniform diameter of between about 1 and 100 millimeters in a natural, as-molded state and may inflate to a diameter of about 10 to 1000 millimeters in an expanded state (as measured at the location of the greatest diameter).

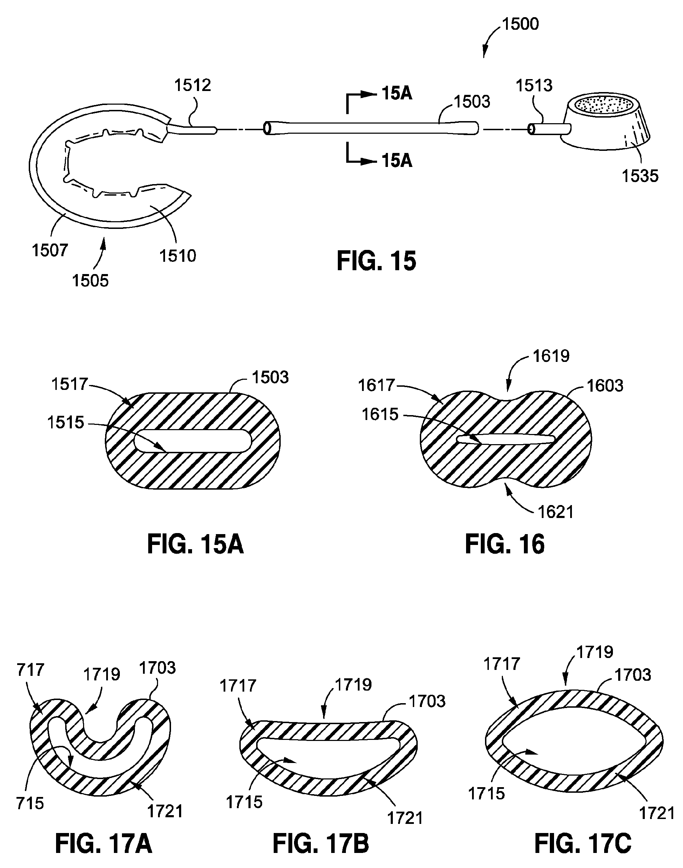

FIG. 15 illustrates a compliant gastric banding system **1500** having a compliant reservoir **1503.** The compliant gastric banding system **1500** may further include a gastric band **1505** having a ring **1507** and an inflatable portion **1510** fluidly coupled to an access port **1535** via the reservoir **1503.** As shown, the reservoir **1503** may have a flattened tube-like appearance and may replace a balloon-type reservoir (e.g., reservoir/balloon **910, 1010, 1110, 1250**) as a compliant component in any gastric banding system (e.g., gastric banding systems **900, 1000, 1100, 1200**). As shown, the reservoir **1503** may appear to have a flattened, tubular structure and may be sized to expand and hold fluid displaced from an adjacent non-compliant reservoir (e.g., inflatable portion **1510**). The reservoir **1503** may be a fluid conduit for fluid transfer between the gastric band **1505** and the access port **1535.** In one embodiment, the reservoir **1503** may be designed to be the compliant component and may function to allow for expansion so that unwanted fluid may move from a coupled stiffer tube (e.g., tube **1512** or **1513**), thus reducing the amount of fluid in the stiffer tube (e.g., the tube **1512** or **1513**), for a short period of time, when pressure is applied to the fluid within the stiffer tube (e.g., the tube **1512** or **1513**).

FIG. 15A is a cross-sectional view of the reservoir **1503** of FIG. 15 and illustrates that a cross-sectional area **1515** within the reservoir **1503** where the fluid flows is continuous and smaller than a cross-sectional area **1517** of the reservoir **1503** itself. In addition to providing a unique, expandable shape profiled over a continuous length, improved performance may be achieved through reduction of the effects of the external forces on fluid within an adjacent, non-compliant component.

The reservoir **1503** of FIG. 15 may be replaced by a reservoir **1603** of FIG. 16. A cross-section of the tube **1603** is illustrated in FIG. 16. As shown, a cross-sectional area **1615** within the reservoir **1603** where the fluid flows is continuous and smaller than a cross-sectional area **1617** of the tube reservoir itself. In addition to providing a unique, expandable shape profiled over a continuous length, improved performance may be achieved through reduction of the effects of the external forces on fluid within an adjacent, non-compliant component. As compared to the reservoir **1503** of FIG. 15, the tube **1603** may have a smaller cross-sectional area **1617** where fluid may flow such that, an increase in fluid may cause indented portions **1619** and **1621** to expand.

The reservoir **1503** of FIG. 15 may be replaced with a reservoir **1703** of FIG. 17A. A cross-section of the reservoir **1703** is illustrated in FIG. 17A. As shown, a cross-sectional area **1715** within the reservoir **1703** where the fluid flows is continuous and smaller than a cross-sectional area **1717** of the reservoir **1703** itself. In addition to providing a unique, expandable shape profiled over a continuous length, improved performance may be achieved through reduction of the effects of the external forces on fluid within an adjacent, non-compliant component. As compared to the reservoir **1503** of FIG. 15, the reservoir **1703** may include a U-shaped tubular structure sized to expand such that a top portion **1719** may be configured to expand from a U-shaped curve to a flatter curve (as shown in FIG. 17B) when more fluid is added to the reservoir **1703.** When even more fluid is added to the reservoir **1703** (e.g., beyond a certain volume threshold), the flatter curve may protrude outward (as shown in FIG. 17C). In one embodiment, as the top portion **1719** changes from the U-shaped curve to the flatter curve to the protruded curve as fluid is added, the bottom portion **1721** may expand longitudinally (e.g., straighten out) as shown in FIGS. 17B and 17C.

The reservoirs **1503, 1603** and **1703** may each be constructed out of a polymer such as silicon, in various durometers. By controlling the durometer of the constructed material, the expansion rates as fluid is added to the reservoirs **1503, 1603** and **1703** may be controlled.

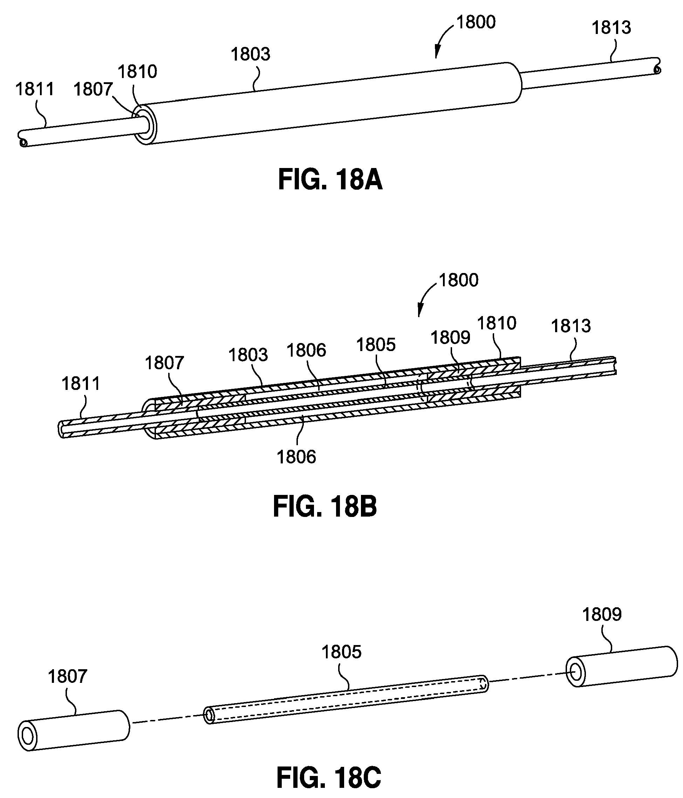

FIG. 18A illustrates a compliant tube-on-tube reservoir system **1800.** The reservoir system **1800** may replace a balloon-type reservoir (e.g., reservoir/balloon **910, 1010, 1110, 1250**) or another tube-like reservoir (e.g., reservoir **1303**) as a compliant component for any gastric banding system (e.g., gastric banding systems **900, 1000, 1100, 1200, 1300**). In one embodiment, the compliant tube-on-tube reservoir system **1800** may include a tube **1803** having an outer tube **1810** and joining portions (first joining portion **1807** shown here) configured to couple the tube **1803** (including an inner compliant tube **1805,** shown in FIG. 18B) with non-compliant tube portions **1811** and **1813.** FIG. 18B illustrates a cross sectional view of the system **1800,** including the internal structure not shown in FIG. 18A. The inner compliant tube **1805** may enlarge when fluid is introduced into the cavity of the inner compliant tube **1805.** The inner compliant tube **1805** may include a diameter substantially equivalent to the diameters of the non-compliant tube portions **1811** and **1813**. The inner compliant tube **1805** may be coupled to the non-compliant tube portions **1811** and **1813** by the first joining portion **1807** and a second joining portion **1809.** As shown, the joining portions **1807** and **1809** may be configured to have an inner circumference sized to fit both the inner compliant tube **1805** and the non-compliant tube portions **1811** and **1813.** In one embodiment, the joining portions **1807** and **1809** do not contact one another, but instead form a gap **1806** between them. The gap **1806** may further be defined by the outer surface of the inner compliant tube **1805** and the inner surface of the outer tube **1810.** The gap **1806** may serve as a sealed space for expansion of the inner compliant tube **1805** and to provide a buffer when the outer tube **1810** is compressed. The outer tube **1810** may, in one embodiment, be rigid, and may be further sized to fit the joining portions **1807** and **1809** within its internal cavity. Generally, as a patient's body presses on the non-compliant tube portions **1811** and **1813,** and/or on the outer tube **1810,** kinks, bending, or compression which disrupts fluid flow may be prevented.

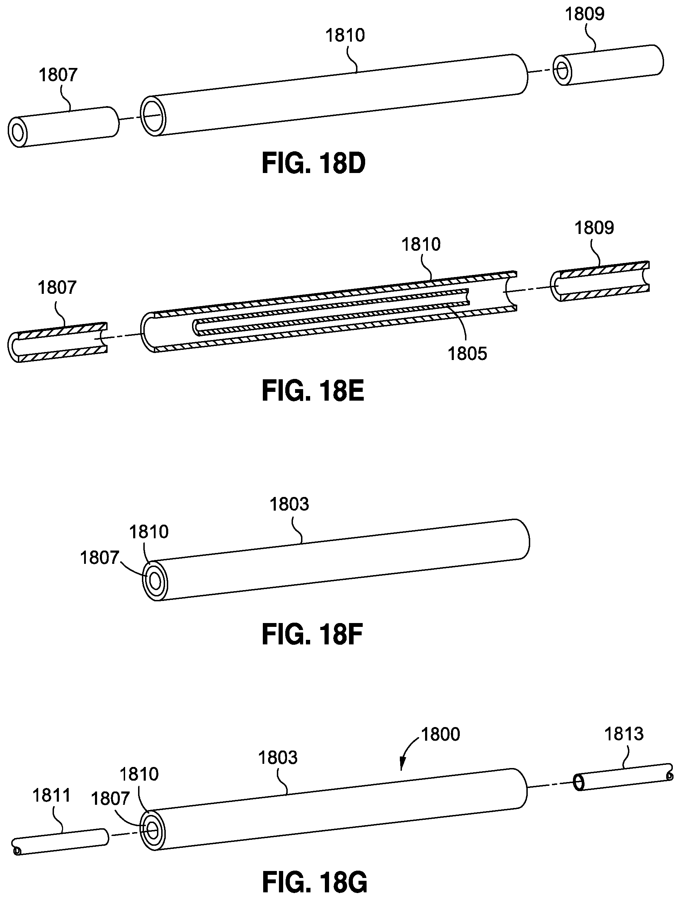

FIGS. 18C - 18G illustrate a method of constructing the system **1800** of FIGS. 18A and 18B. As shown in FIG. 18C, the inner compliant tube **1805** may be inserted into the first joining portion **1807** and the second joining portion **1809.** In one embodiment, an adhesive may be used to attach the surfaces. However, other attachment methods may be practiced without the use of any adhesive (e.g., where the inner compliant tube **1805** is sized to fit tightly into the cavity of the joining portions **1807** and **1809**). FIG. 18D illustrates the outer tube **1810** overlaying the joining portions **1807** and **1809.** FIG. 18E illustrates a deconstructed view of the placement of the inner compliant tube **1805** with respect to the outer tube **1810.** FIG. 18F illustrates the compliant tube **1803** as fully constructed. FIG. 18G illustrates the insertion of the two non-compliant tube portions **1811** and **1813** into the compliant tube **1803** to complete the tube-on-tube reservoir system **1800.**

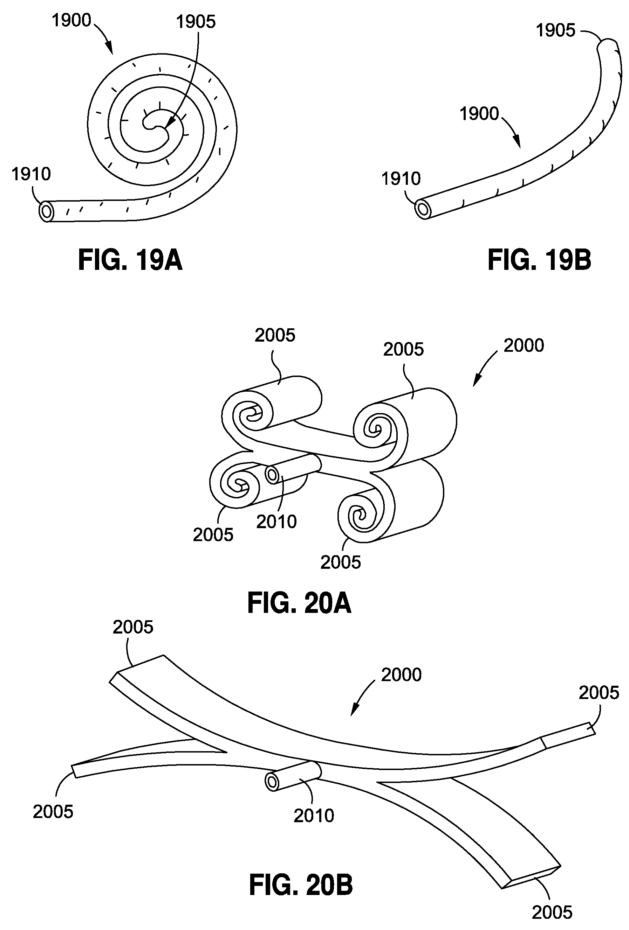

As discussed above with respect to FIGS. 5 and 6, port compliant components **516** and **616** may be used in accordance with various embodiments, such that when the pressure in the inflatable portion **510, 610** exceeds a predetermined pressure, the port compliant component **516** and **616** may expand to receive an amount of fluid from the inflatable portion **510, 610,** the tubing **503, 603,** and/or the access port **535, 635,** and/or to reduce the constriction formed by the gastric band **505, 605.** FIGS. 19A-19B, 20A-20B and 21A-21C illustrate additional compliant reservoir systems **1900, 2000** and **2100,** respectively, that may be implemented as port compliant components **516** and **616.**

The compliant reservoir systems **1900, 2000** and **2100** may be constructed such that a portion of their compliance comes from a conformational change or a shape-change in the systems **1900, 2000** and **2100.** For example, as the pressure inside the reservoir systems **1900, 2000** and **2100** increases, one or more portions may move to a different location or change to a different state. More particularly, the systems **1900, 2000** and **2100** take on a different shape in a pressurized state.

With respect to FIG. 19A, the compliant reservoir system **1900** may be "coiled" in a precurved shape as shown in an unpressurized state. As the pressure increases inside the system **1900** (e.g., as fluid flows into the system **1900** through a distal end **1910**), a proximal end **1905** of the coiled, precurved shape may begin to "uncoil" and straighten out as the system **1900** expands from a flat tube to a more rounded tube, as shown in FIG. 19B. As the pressure is removed, the system **1900** may reform and revert into the coil-like shape due to stresses in the material.

FIG. 20A illustrates a compliant reservoir system **2000** having a distal end **2010** and "coiled wings" **2005.** As shown, system **2000** may have four "coiled wings" but any number of "coiled wings" may be implemented. The "coiled wings" in FIG. 20A are shown in an unpressurized state. As the pressure increases inside the system **2000** (e.g., as fluid flows into the system **2000** through a distal end **2010**), each of the "coiled wings" **2005** may begin to uncoil and straighten out as the system **2000** expands when fluid is increasingly added causing the pressure. FIG. 20B illustrates the system **2000** in a pressurized state. Here, the "coiled wings" are straightened out and no longer in the coiled position. As pressure is removed, the system **2000** may reform and revert into the coil-like shape due to stresses in the material.

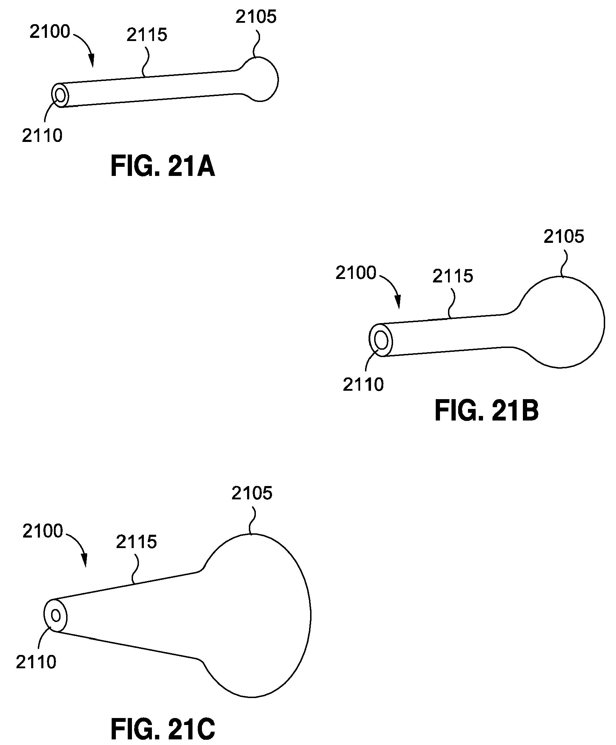

FIG. 21A illustrates a compliant reservoir system **2100** having a distal opening **2110,** a body portion **2115** and a bulged proximal end **2105.** As the pressure increases inside the system **2100** (e.g., as fluid flows into the system **2100** through the distal end **2110**), the bulged proximal end **2105** may enlarge. FIG. 21B illustrates the system **2100** in a pressurized state with an enlarged bulged proximal end **2105** storing the fluid. Here, as the fluid collects in the bulged proximal end **2105,** the bulged proximal end **2105** may further increase in size. In one embodiment, the body portion **2115** may remain substantially the same size or increase slightly in response to the introduction of fluid. FIG. 21C illustrates the system **2100** when a large amount of fluid is introduced into the system **2100,** thereby causing the preferential inflation of the bulged proximal end **2105.** However, as shown in FIG. 21C, other portions of the system **2100** may also incrementally increase in size such as the body portion **2115.** As pressure is removed, the system **2100** may revert to the shape of FIG. 21A due to stresses in the material.

In addition to, or as an alternative to reservoirs with different shapes and structures (e.g., compliant reservoir system **1900, 2000** and **2100**), embodiments of compliant reservoirs may include internal structures such as springs, cages and/or rings.

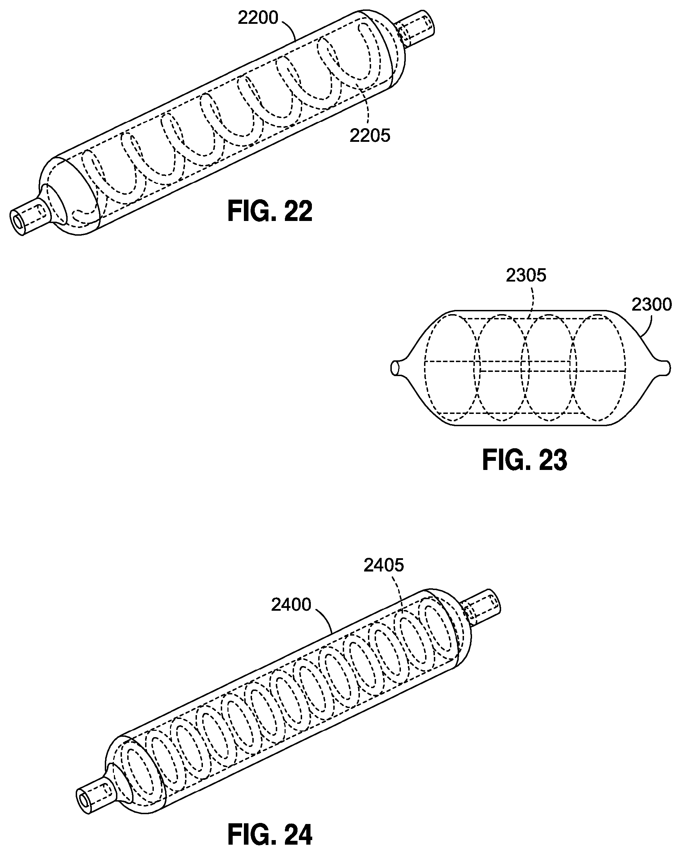

FIG. 22 illustrates a compliant reservoir **2200** which may be integrated into the tubing (e.g., tubing **403** or **503**), added as an additional compliant component and/or attached to the access port (e.g., compliant component **516**). As shown in FIG. 22, the compliant reservoir **2200** may include a spring **2205** which provides a skeletal structure. As the patient's body presses on the compliant reservoir **2200,** the spring **2205** prevents the compliant reservoir **2200** from being kinked, bent or compressed in a way that disrupts flow of fluid or causes unwanted fluid to move into the inflatable portions (e.g., inflatable portions **510** and **610**). In one embodiment, the spring **2205** may provide the compliant reservoir **2200** properties including high radial stiffness and low axial stiffness. The radial stiffness allows the compliant reservoir **2200** to support weight applied externally while the low axial stiffness provides for volumetric expansion of the compliant reservoir **2200** internally. In addition, the spring **2205** may provide internal support to resist loading from body tissues without an increase in pressure inside the compliant reservoir **2200.** In one embodiment, the spring **2205** and the compliant reservoir **2200** may be configured to increase pressure relative to the volume displaced.

FIG. 23 illustrates a compliant reservoir **2300** having an internal structure in the form of a cage **2305.** The compliant reservoir **2300** may be integrated into a tubing (e.g., tubing **403** or **503**), added as an additional compliant component and/or attached to the access port (e.g., compliant component **516**). As shown in FIG. 23, the compliant reservoir **2300** may include the internal cage **2305,** which provides the compliant reservoir with a skeletal structure. As the patient's body presses on the compliant reservoir **2300,** the rings and bars of the cage **2305** may prevent the compliant reservoir **2300** from being kinked, bent or compressed in a way that disrupts the flow of fluid or causes unwanted fluid to move into the inflatable portions (e.g., inflatable portions **510** and **610**). The cage **2305** may provide the compliant reservoir **2300** high radial stiffness and low axial stiffness. The radial stiffness may allow the compliant reservoir **2300** to support weight applied externally while the low axial stiffness provides for volumetric expansion of the compliant reservoir **2300** internally. In addition, the cage **2305** may provide internal support to resist loading from body tissues without an increase in pressure inside the compliant reservoir **2300.** The cage **2305** and the compliant reservoir **2300** may be configured to increase pressure relative to the volume displaced.

FIG. 24 illustrates a compliant reservoir **2400** having an internal structure in the form of parallel rings **2405.** The compliant reservoir **2400** may be integrated into a tubing (e.g., tubing **403** or **503**), added as an additional compliant component and/or attached to the access port (e.g., compliant component **516**). As shown in FIG. 24, the compliant reservoir **2400** may include the parallel rings **2405,** which provides the compliant reservoir **2400** with a skeletal structure. In one example, the parallel rings **2405** may be joined together. As the patient's body presses on the compliant reservoir **2400,** the rings **2405** may prevent the compliant reservoir **2400** from being kinked, bent or compressed in a way that disrupts flow of fluid or causes unwanted fluid to move into the inflatable portions (e.g., inflatable portions **510** and **610**). The rings **2405** may provide the compliant reservoir **2400** high radial stiffness and low axial stiffness. The radial stiffness may allow the compliant reservoir **2400** to support weight applied externally while the low axial stiffness provides for volumetric expansion of the compliant reservoir **2400** internally. In addition, the rings **2405** may provide internal support to resist loading from body tissues without an increase in pressure inside the compliant reservoir **2400.** The rings **2405** and the compliant reservoir **2400** may be configured to increase pressure relative to the volume displaced.

While the internal structures (e.g., the spring **2205,** the cage **2305** and the parallel rings **2405**) of FIGS. 22, 23 and 24 may have different configurations, they may provide for different or same pressures. In one embodiment, the spring **2205,** the cage **2305** and the parallel rings **2405** may be made of metals or polymers such as stainless steel, titanium, nitinol, PEEK, ultem, delrin, polycarbonate, polysulfone, among other materials. Materials used may further be combined to provide the desired properties. With respect to construction, the spring **2205,** the cage **2305** and the parallel rings **2405** may be over-molded and/or contiguous with the compliant reservoir (e.g., compliant reservoirs **2200, 2300** and **2400**).

In addition, or as an alternative to the internal structures of FIGS. 22-24, external structures may be utilized to protect a compliant reservoir from being kinked, bent or compressed in a way that disrupts flow of fluid or causes unwanted fluid to move into the inflatable portions (e.g., inflatable portions **510** and **610**).

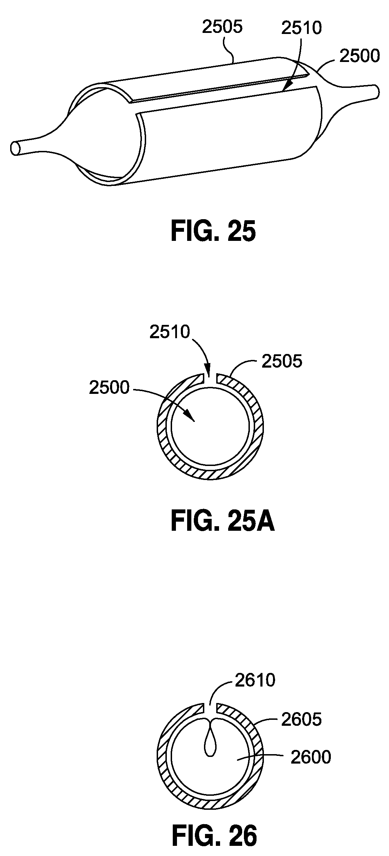

FIG. 25 illustrates a reservoir **2500** with an outer protective layer **2505.** The outer protective layer **2505** may be an exoskeleton configured to wrap axially about the reservoir **2500.** The outer protective layer **2505** may protect the reservoir **2500** from external forces. As shown, the outer protective layer **2505** may include a hinge cut **2510** causing a gap within the outer protective layer **2505.** The hinge cut **2510** may allow the outer protective layer **2505** to be deformable, thereby allowing the reservoir **2500** to be compliant even after the fluid-volume level of the reservoir **2500** causes the outer diameter of the reservoir **2500** to exceed the inner diameter of the outer protective layer **2505.** In other words, the hinge cut **2510** allows the outer protective layer **2505** to expand (increasing the width of the gap), which in turn allows the reservoir **2500** to continue to expand as it receives additional fluid. FIG. 25A is a cross-sectional view of the reservoir **2500** having the separate, overlaying outer protective layer **2505.** In one embodiment, the reservoir **2500** may be attached or integrated with the outer protective layer **2505** (e.g., by using an adhesive). The outer protective layer **2505** may further be used with reservoirs of different shapes. For example, FIG. 26 illustrates a cross sectional view of an outer protective layer **2605** surrounding a u-shaped reservoir **2600.** As shown, the outer protective layer **2605** may include an optional hinge cut **2610.**

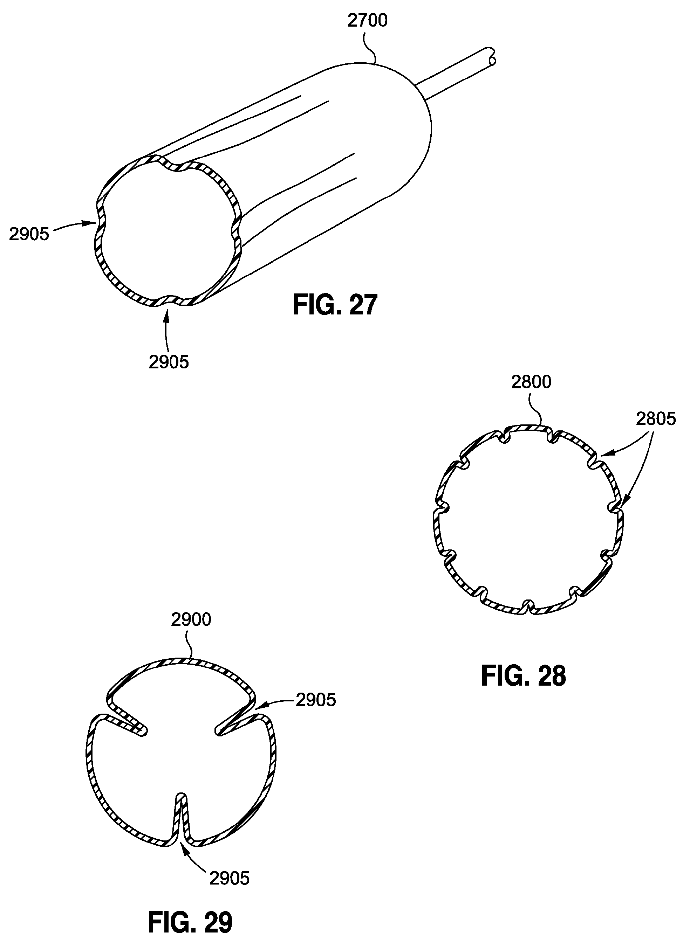

As discussed herein, the reservoirs (e.g., reservoir **2500** of FIG. 25 and reservoir **2600** of FIG. 26, among other embodiments of reservoirs) may be shaped differently. In addition and/or alternatively, the reservoirs may include depressions, pleatings or longitudinal structures along an outer circumferential perimeter. For example, FIG. 27 illustrates a reservoir **2700** having four patterned depressions **2705** which may buckle (inward) in a reproducible manner when suction is applied to the reservoir **2700** by a surgical trocar (not shown). However, other depression patterns are also possible. For example, FIG. 28 illustrates a cross section of a reservoir **2800** having eleven patterned depressions **2805.** FIG. 29 illustrates a cross section of a reservoir **2900** having three patterned depressions **2905.** While FIGS. 27-29 illustrate reservoirs **2700, 2800** and **2900** having equally spaced depressions of different depths, non-uniformly spaced depressions are also possible for the purpose of guiding deflation.

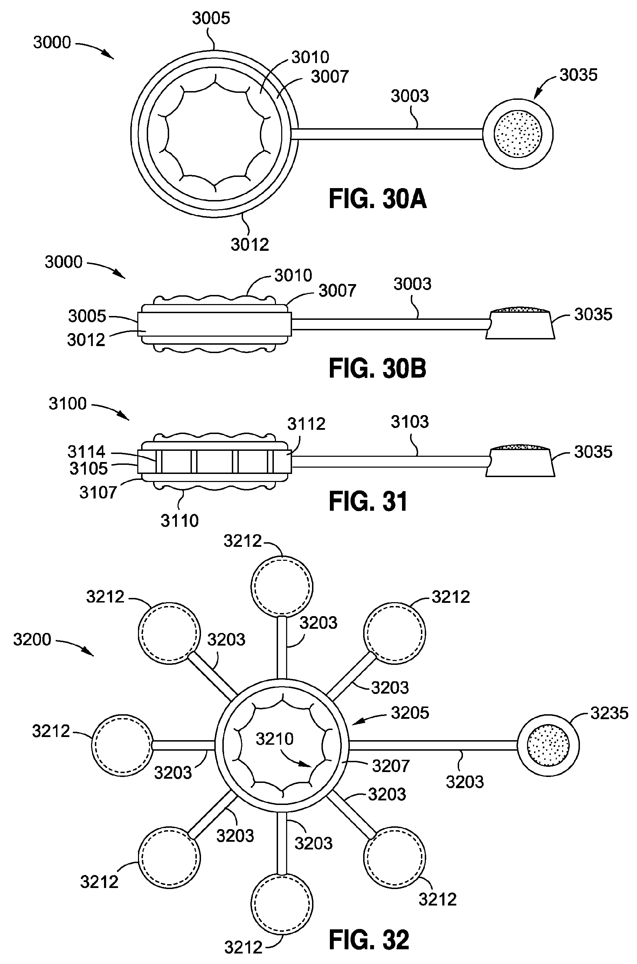

In addition and/or as an alternative to different reservoir shapes, a reservoir may be oriented in different ways. FIG. 30A illustrates a compliant reservoir system **3000** which may include a gastric band **3005** having an inflatable portion **3010,** a ring **3007** and a compliant portion **3012.** The gastric band **3005** may be fluidly coupled to a tubing **3003** and an access port **3035.** As shown, the compliant reservoir **3012** may be oriented circumferentially about the ring **3007.** The compliant reservoir **3012** may be in fluid communication (not shown) with the inflatable portion **3010** through a hole or other path (e.g., extending across the ring **3007**). Accordingly, when a large bolus is swallowed by the patient, the fluid within the inflatable portion **3010** may be dispersed and may flow into the compliant reservoir **3012,** thereby allowing the large bolus to pass through. Similar to the reservoirs **910, 1010, 1110** and **1250** of FIGS. 9-12, the compliant reservoir **3012** may be constructed of biocompatible, elastomeric material. FIG. 30B is a side view of the compliant reservoir system **3000** of FIG. 30A.

Similar to the compliant reservoir system **3000,** a compliant reservoir system **3100** may include a gastric band **3105** having an inflatable portion **3110,** a ring **3107** and a compliant portion **3112.** The gastric band **3105** may be fluidly coupled to a tubing **3103** and an access port **3135.** However, as shown in FIG. 31, the compliant reservoir system **3100** may further include vertical molding portions **3114.** The vertical molding portions **3114** may be equally spaced apart about the outer circumference of the compliant reservoir **3112** and may be attached to the ring **3107,** functioning to provide the compliant reservoir **3112** with structural support. In this manner, the compliant reservoir **3112** may be integrated with the ring **3107.**

FIG. 32 illustrates a compliant reservoir system **3200** having a gastric band **3205** with an inflatable portion **3210** and a ring **3207.** The gastric band **3205** may in fluid communication with an access port **3235** and a plurality of compliant reservoirs **3212.** As shown, seven reservoirs **3212** may be oriented radially around the gastric band **3205** and may be coupled to the gastric band **3205** via corresponding tubing **3203.** While each reservoir **3212** is shown to be substantially equidistant from the gastric band **3205,** alternative geometries are possible (e.g., one or more of the reservoirs **3212** may be closer to the gastric band **3205** via shorting tubing). In addition, the number of reservoirs **3212** may vary (e.g., any number of reservoirs **3212** between one and twenty inclusive, may be included). These reservoirs **3212** may function similarly as the reservoirs **3012** and **3112** of FIGS. 30A and 31, respectively. For example, when a large bolus is swallowed by the patient, the fluid within the inflatable portion **3210** may be dispersed and may flow into any or all of the compliant reservoirs **3212,** thereby allowing the large bolus to pass through. In addition, the reservoir **3212** may be constructed out of any one or a combination of biocompatible, elastomeric material.

Reservoirs of different types, configurations, and orientations having been discussed, attention will now be turned to the fill substance of the gastric banding systems. The fill substances discussed herein may be applicable to any gastric banding system, including any of the compliant reservoir systems discussed above (e.g., systems **900, 1000, 1100,** and **1200**).

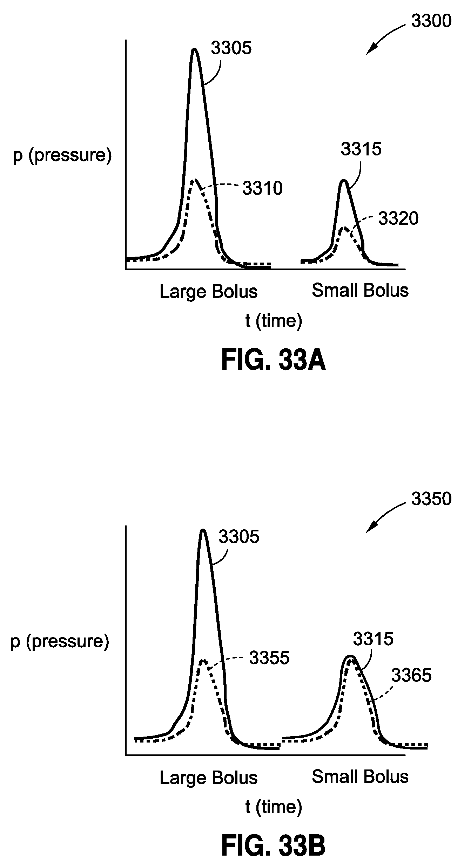

FIG. 33A illustrates a pressure-time graph **3300** comparing a compliant reservoir system utilizing saline or another fluid with a non-compliant gastric banding system. As shown, when a large bolus passes through the constriction of a non-compliant gastric banding system, a large spike in pressure **3305** is exerted whereas the same large bolus passing through the constriction of a compliant reservoir system utilizing saline may result in a lower pressure spike **3310.** In addition, a higher pressure spike **3315** may occur when a small bolus passes through the constriction of the non-compliant gastric banding system, and a lower pressure spike **3320** may occur when the same small bolus passes through the constriction of the compliant reservoir system utilizing saline.

While FIG. 33A illustrates the benefits of a compliant reservoir system utilizing saline for situations where a large bolus is present over a non-compliant gastric banding system, further improvement may be possible with respect to small bolus reactions.

FIG. 33B illustrates a pressure-time graph **3350.** The pressure-time spikes **3305** and **3315** correspond to a large bolus and a small bolus, respectively, passing through a constriction formed by a non-compliant gastric banding system. In comparison, the pressure spikes **3355** and **3365** correspond to a large bolus and a small bolus, respectively, passing through a constriction formed by a compliant reservoir system utilizing non-saline substances. More particularly, when a large bolus is introduced, the compliant reservoir system utilizing non-saline substances may perform substantially similar to a compliant reservoir system utilizing saline substances (e.g., comparing pressure spike **3310** of FIG. 33A and pressure spike **3355** of FIG. 33B). However, when a small bolus is introduced, the compliant reservoir system utilizing non-saline substances may perform substantially similar to a non-compliant reservoir system (e.g., comparing pressure spike **3315** of FIG. 33A and pressure spike **3365** of FIG. 33B). The response to large boluses and small boluses as illustrated in pressure spike **3355** and **3365,** respectively, may be preferable for providing overall improved efficacy.

These non-saline substances which provide for the preferred pressure spikes **3315** and **3365** of FIG. 33B may include a pseudoplastic fluid, a Bingham plastic, and the like.

A pseudoplastic fluid may be utilized to fill a gastric banding system (e.g., gastric banding system **500, 600**). A pseudoplastic fluid may exhibit a decrease in viscosity under increases in the shear rate. Accordingly, only a slight increase in pressure would lead to relatively low shear rates (and relatively high viscosity) while a more substantial increase in pressure would lead to relatively high shear rates (and relatively low viscosity). In this manner, small to medium increases in pressure within the gastric banding system (e.g., gastric banding system **500, 600**) will yield results similar to existing non-compliant gastric banding systems, while under higher pressure spikes, the pseudoplastic fluid (due to its lower viscosity at higher pressures) would flow out of the inflatable portions and into a reservoir, curbing the intensity of the pressure spike. Such large pressure spike may occur, for example, when a large bolus is attempting to pass through a constriction of the non-compliant gastric banding system. Once the pressure minimizes and the baseline pressure in the gastric banding system is re-established, the pseudoplastic fluid may gradually return to the inflatable portions.

A Bingham plastic may be utilized as the fluid within the gastric banding system (e.g., gastric banding systems **500, 600**). The Bingham plastic may be a material which does not flow until a minimum yield stress within the fluid is reached. In other words, prior to reaching the minimum yield stress, the Bingham plastic acts as a solid. However, once the minimum yield stress is reached, the Bingham plastic acts a fluid and may flow from the inflatable portions (e.g., the inflatable portion **510, 610**) to a fluidly-coupled reservoirs (e.g., the reservoir **514, 614**). Once the pressure minimizes and the baseline pressure is re-established, the Bingham plastic may flow again and may return to the inflatable portions (e.g., inflatable portions **510, 610**)**.**

In addition to pseudoplastic fluid, the Bingham plastic and the like, other materials may be used to fill any gastric banding system. Moreover, other configurations, including addition or removing various components of the gastric banding system, may be desirable when other fill substances are considered.

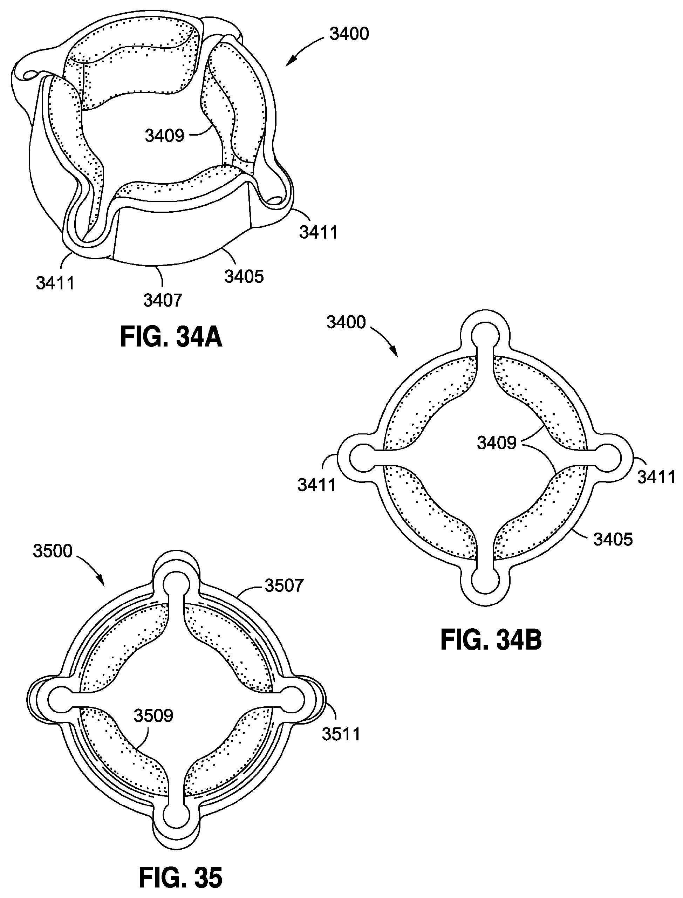

FIG. 34A illustrates a perspective view of a gastric band system **3400** having a different fill material than saline. The gastric band system **3400** may form a circumference about an upper stomach region of a patient and may provide pressure on the patient's stomach to induce satiety. Here, the gastric band system **3400** may include a gastric band **3405** having an outer ring **3407,** cushions **3409** and one or more hinges **3411.** The cushions **3409** may be filled with a gel and/or other substances. Alternatively or in addition, the cushions **3409** may be low durometer foam pads (e.g., less than 40 durometers). The gastric band **3405** may be self-contained and might not involve fluid systems having components such as tubing and access ports. As shown, the gastric band **3405** may include four cushions **3409** separated by four hinges **3411.** However, additional cushions **3409** and/or hinges **3411** may be added, or one or more cushions **3409** and/or hinges **3411** may be removed. The hinges **3411** and the outer ring **3407** may be constructed out of a plastic or other polymer having dimensional stability, low creep and relatively high modulus of elasticity such that it may operate to function as a torsion spring. For example, a PEEK or polysufone material may be utilized, among other materials. Alternatively, the hinges **3411** may be constructed out of metal (e.g., stainless steel, titanium, etc.). In one or more embodiments, when the hinge **3411** is constructed out of a metal, a silicone rubber may encapsulate the hinge **3411.** Regardless of the construction of the hinge **3411,** each may function as a torsion spring generating a substantially constant force on the patient's stomach even when the inside diameter of the gastric band **3405** increases due to the passage of a large bolus of food. For example, as the radial expansion of the gastric band **3405** occurs as caused by the passing of the large bolus of food, an increase in the lever arm of the hinges **3411** partially opposes (and thus cancels out) the effect of the increasing deflection, thereby resulting in a constant force.

FIG. 34B illustrates a top view of the gastric banding system **3400** of FIG. 34A. As shown, the hinges **3411** may be integrated with the outer ring **3407** and the cushions **3409** may have a length substantially spanning a non-hinge portion of the outer ring **3407.** The gastric banding system **3400** of FIGS. 34A and 34B may include a tunable hoop spring rate and may be customized to have an initial diameter for a given patient.

FIG. 35 illustrates a gas-filled gastric banding system 3500. While similar in appearance to the gastric banding system 3400 of FIGS. 34A and 34B, and including features like one or more hinges **3511** and one or more ring portions **3507**, one difference between the gas-filled gastric banding system **3500** and the gastric banding system **3400** is that the cushions **3509** may be constructed out of different materials and may be filled with one or more gases including carbon dioxide, nitrogen, among others. By utilizing the gas-filled cushions **3509**, lower radial stiffness may be achieved, thereby reducing discomfort for the patient when a large bolus attempts to pass through the constriction. A suitable gas-impermeable membrane may be used in constructing the cushions **3509** to prevent the gas from leaking out. For example, a silicone rubber may be used. In addition, coatings to further prevent gas leakage such as a diamond-like carbon, titanium nitrite, parylene, aclar, among other gas impermeable substances, and combinations thereof may be applied to the silicone rubber. In one embodiment, the gastric banding system **3500** might not require a traditional access port for occasional adjustment. Instead, an adjustment just prior to implantation may provide a constant pressure configured to serve the constriction and/or the neural stimulation function properly while large boluses of food are able to pass through due to the compressibility of the gas-filled gastric banding system **3500**.

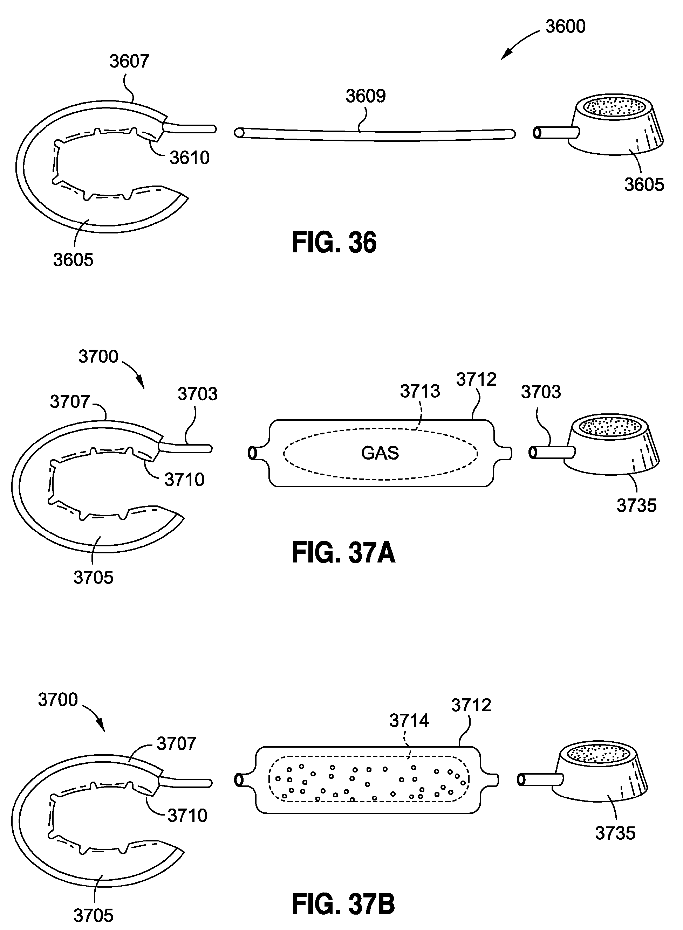

The use of gas as a fill substance is not limited to the system of FIG. 35. For example, as shown in FIG. 36, a gastric banding system **3600** may include a gastric band **3605** having a ring **3607** and an inflatable portion **3610** connected to a tube **3609** and an access port **3635**. However, the inflatable portion **3610**, the tube **3609** and the access port **3635** may all be constructed out of gas impermeable substances such as silicone rubber and/or coated with gas impermeable substances including, but not limited to, a diamond-like carbon, titanium nitrite, parylene, aclar, among other gas impermeable substances and combinations of substances.

A hybrid gas-saline gastric banding system **3700** is illustrated in FIG. 37A. As shown, the hybrid gas-saline gastric banding system **3700** may include a gastric band **3705** having a ring **3707** and an inflatable portion **3710**. The gastric band **3705** may be in fluid communication with a reservoir **3712** and an access port **3735** via tubing **3703**. The gastric banding system **3700** may be filled with a standard saline solution. However, a further encapsulated member **3713** may also be present within the hybrid gas-saline gastric banding system **3700**. The encapsulated member **3713** may be a balloon filled with a gas such as carbon dioxide, nitrogen, and the like. The encapsulated member **3713** may function to further provide flexibility and/or otherwise lower the radial stiffness within the hybrid gas-saline gastric banding system **3700** by moving from the inflatable portion **3710** to the reservoir **3712** when a large bolus is attempting to pass through the constriction of the gastric band **3705**. In one embodiment, the encapsulated member **3713** is within the fluid path and may travel to any component of the gastric banding system **3700** in response to fluid movement through, e.g., the tubing **3703**. In one embodiment, the encapsulated member **3713** may be configured to be located within or traverse back into the inflatable portion **3710** when a large bolus is not attempting to pass through (e.g., when the hybrid gas-saline gastric banding system **3700** is in an equilibrium state).

The encapsulated member **3713** may be constructed out of silicone rubber and may be coated with one or more materials to enhance the ability of the silicone rubber to be gas impermeable thereby preventing the encapsulated member **3713** from leaking.

The encapsulated member **3713** might not be designed to not fit through the tubing **3703** and traverse between the different components of the hybrid gas-saline gastric banding system **3700**. In other words, the encapsulated member **3713** may permanently reside in any one of the components, e.g., the reservoir **3712**.

FIG. 37B illustrates the hybrid gas-saline gastric banding system **3700** utilizing a different encapsulated member **3714** in place of the encapsulated member **3713** of FIG. 37A. The encapsulated member **3714** may have the functionality similar to the encapsulated member **3713** of FIG. 37A, but may be constructed out of a closed cell foam instead of gas.

Modifications may be further made to the hybrid gas-saline gastric banding system **3700**. For example, other compliant portions may be added. Alternatively and/or in addition, in one embodiment, the hybrid gas-saline gastric banding system **3700** as discussed above with respect to FIGS. 37A and 37B may be further modified to eliminate the access port **3735** leaving only the gastric band **3705**, the reservoir **3712** and one of the encapsulated members **3737** or **3738** (not shown). Eliminating the access port **3735** renders the hybrid gas-saline gastric banding system **3700** self-adjusting and may provide constant pressure on the stoma of the patient.

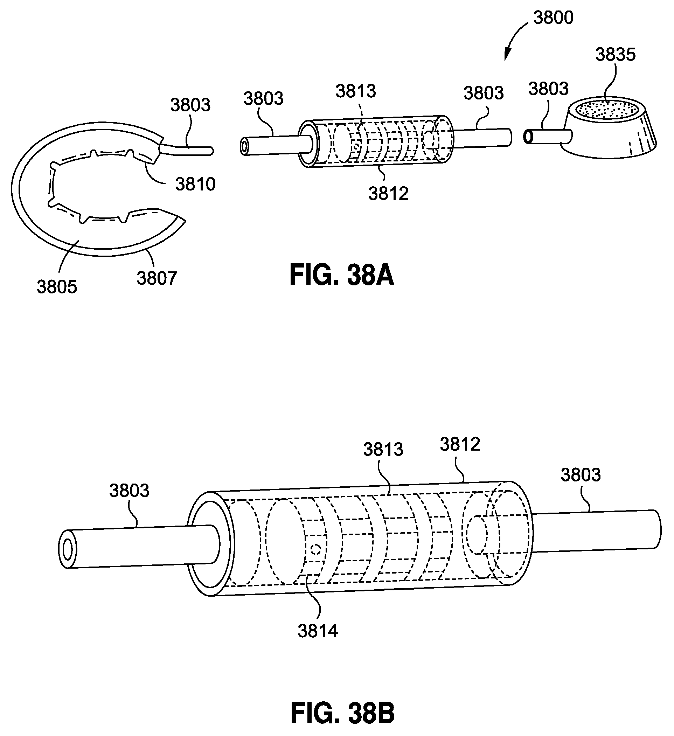

FIG. 38A illustrates another hybrid gas-saline gastric banding system **3800**. As shown, the hybrid gas-saline gastric banding system **3800** may include a gastric band **3805** having a ring **3807** and an inflatable portion **3810**. The gastric band **3805** may be in fluid communication with a reservoir **3812** and an access port **3835** via tubing **3803**. The gastric banding system **3800** may be filled with a standard saline solution. The reservoir **3812** may be cylindrical in shape and may be constructed out of a silicon rubber or another durable, gas impermeable material. The tubing **3803** may be held in place within the reservoir **3812** by an adhesive layer or patch. In addition, the reservoir **3812** may house a gas spring **3813**. In one embodiment, the gas spring **3813** may be retained in the reservoir **3813** by radial interference (e.g., the gas spring **3813** may be designed to fit tightly within the reservoir **3812** to avoid slippage or other un-intended movement). The gas spring **3813**, while being movably fixed within the reservoir **3812**, may still allow for fluid to pass through by having uneven surfaces (e.g., non-circular geometry which creates gaps and/or openings for fluid to pass through between an outside surface of the gas spring **3813** and an inner diameter of the reservoir **3812**). Generally, the gas spring **3813** may operate to provide volume flexibility and/or low radial stiffness in the gastric banding system **3800**. For example, when a large bolus of food passes through a constriction of the gastric band **3805**, pressure caused by the bolus on the inflatable portion **3810** may cause a transfer of fluid from the inflatable portion **3810** to the reservoir **3812**. One function of the gas spring **3813** is to alleviate the pressure spike caused by the food bolus by moving a piston within the gas spring **3813** and winding the gas spring **3813**. Once the pressure is removed (e.g., the bolus having been passed through the constriction), the gas spring **3813** may unwind and revert back to a non-compressed orientation.

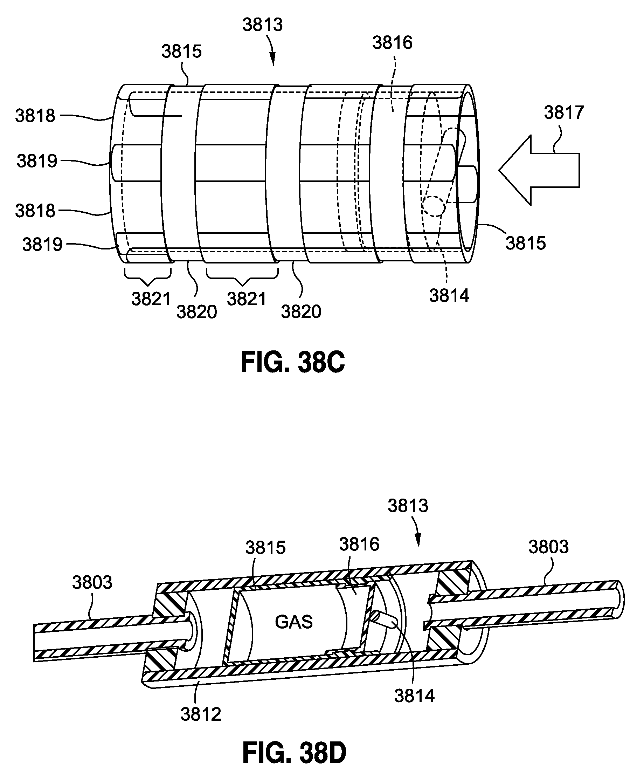

FIG. 38B illustrates the reservoir **3812** and the gas spring **3813** apart from the other portions of the hybrid gas-saline gastric banding system **3800**. FIG. 38C illustrates a further deconstructed view of the gas spring **3813** apart from the reservoir **3812**. As shown in FIG. 38C, a pin **3814** may be fixed in place to the inner diameter of the gas spring housing **3815** thereby limiting the movement of the piston **3816**. Arrow **3817** illustrates a direction of fluid pressure (e.g., caused by a large bolus passing through the constriction of the gastric band **3805**) which may move the piston **3816** and compress the gas within the gas spring housing **3815**. The gas within the gas spring housing **3815** may be sealed between the piston **3816** and compressed when pressure is introduced. The piston **3816** and the inner surface of the gas spring housing **3815** may be formed out of materials with very low coefficient of friction such that the overall friction force does not overly prevent the piston **3816** from moving when a fluid pressure is introduced. The outside surface of the gas spring housing **3815** may include contact segments **3821** and fluid passageway segments **3820** configured in an alternating manner. An outer diameter of any given fluid passageway segment **3820** may be smaller than an outer diameter of any given contact segment **3821**. The contact segments **3821** may further include curved portions **3818** for contacting an inner diameter of the reservoir **3812** of FIG. 38B, thereby holding or fixing the gas spring **3813** in place. The contact segments **3821** may further include flat portions **3819** which might not contact the inner diameter of the reservoir **3812**, thereby allowing fluid flowing through the fluid passageway segment portions **3818** to also flow through the contact segments **3821**. In this manner, fluid within the gastric banding system **3800** may flow from, for example, the gastric band **3805** to the access port **3835**, and vice versa. In one embodiment, the flat portions **3819** and the curved portions **3813** of each contact segment **3821** may alternate in configuration around the circumference of the gas spring housing **3815**.

FIG. 38D is a cross-sectional view of the reservoir **3812** and the gas spring **3813** apart from the other portions of the hybrid gas-saline gastric banding system **3800** to better illustrate the configuration of the components therein. Appropriate materials to construct one or more parts of the gas spring **3813** (e.g., gas spring housing **3815**, piston **3816**, etc.) may include gas-impermeable and/or saline-impermeable substances such as plastics like PTFE.

In addition to and/or as an alternative to a hybrid gas-saline gastric banding system of FIG. 38 having a reservoir (e.g., the reservoir **3812**) functioning as the gas-saline component, other components of the gastric banding system may be the gas-saline component. For example, an access port of any gastric banding system may be the gas-saline component.

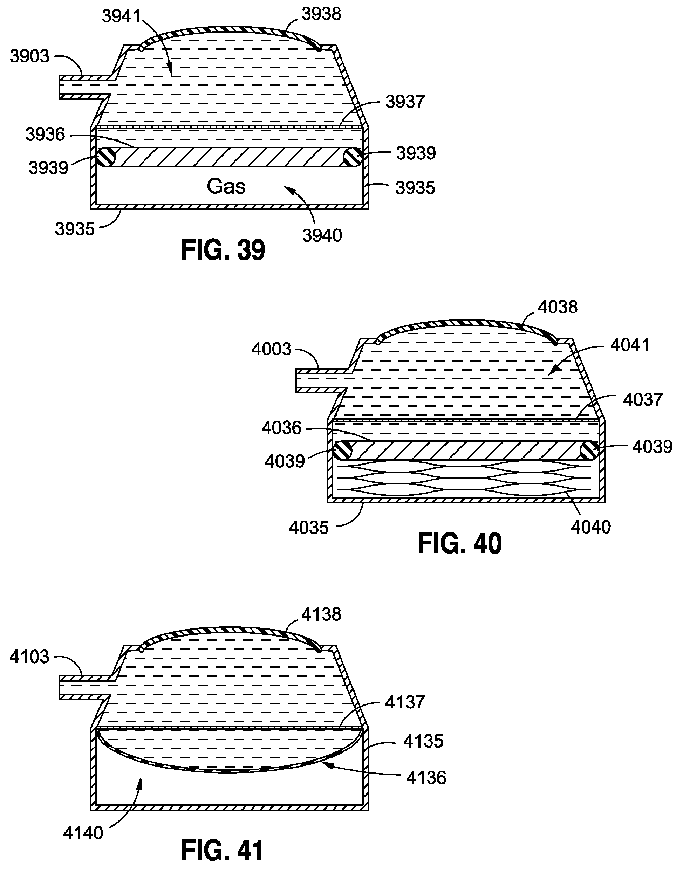

FIGS. 39-41 illustrate various access ports **3935, 4035, 4135.** While the rest of the gastric banding system is not shown in these figures, the access ports **3935**, **4035, 4135** may be in fluid communication with other components of the gastric banding system (e.g., a gastric band and/or a reservoir) via tubes **3903, 4003, 4103,** respectively. For example, access ports **3935, 4003, 4103** may each be the access port **435** or **535.**

Turning to FIG. 39, the access port **3935** may include a movable surface **3936** which may move in response to a pressure change within the gastric banding system. For example, the movable surface **3936** may be a complaint portion that moves to increase the volume of the fluid portion **3941** (and therefore increases compliance) when the pressure increases within the gastric banding system (e.g., in response to a large bolus moving through a constriction of the gastric band). Once the pressure is reduced (e.g., the large bolus passing through the constriction), the movable surface **3936** may return to its original position, thereby decreasing the volume of the fluid portion **3941** of the access port **3935**. The access port **3935** may further include a septum **3938**, a fluid-permeable membrane **3937**, o-rings **3939** and a gas spring portion **3940** filled with a gas **3935**.

Alternatively, the gas spring portion **3940** may be replaced with a wave spring, a cantilever spring, a constant force spring, a coil spring, a leaf spring, a Belleville spring, a hybrid polymer coil-air spring and the like. In addition, a vacuum (not shown) may be incorporated in order to achieve the desired pressure response.

FIG. 40 illustrates an example of an access port **4035** having a wave spring **4040**. The access port **4035** may be in fluid communication with other components of the gastric banding system (e.g., a gastric band and/or a reservoir) via tube **4003**. In one embodiment, the access port **4035** may include a movable surface **4036** which may move in response to a pressure change. For example, the movable surface **4036** may be a complaint portion that moves to increase the volume of the fluid portion **4041** (and therefore increases compliance) in response to a large bolus moving through a constriction of the gastric band (not shown). Once the pressure is reduced (e.g., the large bolus passes through the constriction), the movable surface **4036** may return to its original position thereby decreasing the volume of the fluid portion **4041** of the access port **4035**. The access port **4035** may further include a septum **4038**, a fluid-permeable membrane **4037**, o-rings **4039** and a wave spring **4040**.

The spring (e.g., gas spring **3940** of FIG. 39 or wave spring **4040** of FIG. 40) may be removed as shown in FIG. 41. Here, the moving surface **4136** may comprise a flexible membrane that deforms when subjected to pressures above a certain threshold (which in one example, may be set to approximate the pressure induced when a large bolus is passing through a constriction). Once the pressure falls below the threshold again (e.g., when the large bolus passes through the constriction), the moving surface **4136** may return to its original configuration. The moving surface **4136** may be constructed out of silicone or another biocompatible flexible polymer, among other materials. The moving surface **4136** may be attached to the inner wall of the access port **4135** or may be integrated with a septum **4138** of the access port **4135**.

Each of the access ports **3935, 4035** and **4135** may include a corresponding septum **3938**, **4038** and **4138**. The septum **3938**, **4038** and **4138** may function to allow a penetrating needle to add or remove fluid thereby adjusting the total fluid volume within the corresponding gastric banding system. In addition, the access ports **3935, 4035** and **4135** may include a corresponding fluid-permeable membrane **3937, 4037** and **4137** having a plurality of small openings (not shown) which may be large enough to allow fluid to pass through, but are small enough to prevent the passage of the needle. The fluid-permeable membranes **3937, 4037** and **4137** may be constructed out of a metal, ceramic, carbon, polymer or any combination thereof. Other materials may also be used so long as the material prevents the passage of the needle. The fluid-permeable membranes **3937, 4037** and **4137** may be attached to the inner wall of the corresponding access port **3935, 4035** and **4135**.

In addition, the access ports **3935** and/or **4035** may include a corresponding sealing ring **3939** and **4039** to prevent fluid from entering the spring portions **3940** and **4040**. The sealing rings **3939** and **4039** may be a polymer o-ring, a metal seal ring, a ceramic seal ring, a polymer seal ring and the like.

In addition to using pressure to control the flow of fluid, gas, among other substances within a gastric banding system, specific restriction devices may also be utilized. For example, a one-way valve, a pressure relief valve, orifices, turbulence controllers, sponges, environment-adapting devices, among other restriction devices may control a rate at which a component (e.g., a compliant reservoir) may receive fluid from another component (e.g., a gastric band).

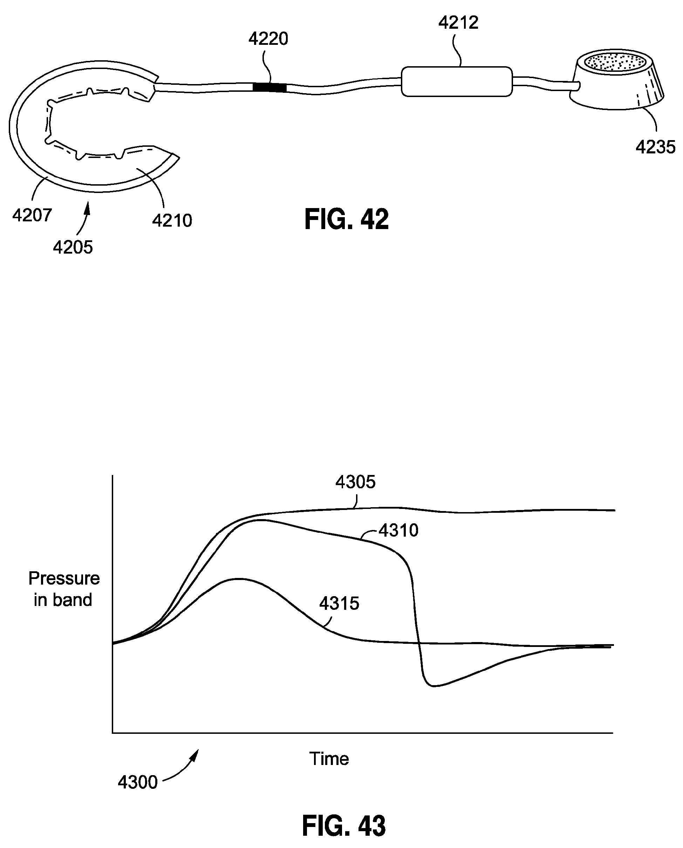

FIG. 42 illustrates a gastric banding system **4200** having a gastric band **4205,** an inflatable portion **4210,** and a ring **4207** in fluid communication with a reservoir **4212** through a restriction device **4220.** Also shown is an access port **4235** in fluid communication with the above-mentioned components of the gastric banding system **4200.** The restriction device **4220** may be electronically controlled, modulated with an external adjustment system or may be a passive restriction device configured to be independent from being controlled by an external tool or device.

In practice, any one (or more) of a plurality of restriction devices (e.g., restriction device **4220**) may be used within the fluid path of the gastric banding system **4200** or any other gastric banding system. Further, additional restriction devices may be added to achieve the desired flow rates. FIG. 43 illustrates a graph **4300** depicting how the time-pressure curve may change when such a restriction device **4220** is used (e.g., an orifice having an inner diameter which limits the flow rate of the fluid in both directions). For comparison, line **4305** is a time-pressure curve corresponding to a standard non-compliant band. As shown, the pressure may sharply rise when a large bolus encounters the constriction formed by the standard non-compliant band may become obstructed. As shown, the pressure may remain high for the duration of the obstruction caused by the bolus, and the patient may experience discomfort for a long period of time. Line **4315** is a time-pressure curve corresponding to a compliant band without a restriction device. As shown, the large bolus of food may cause only a modest increase in pressure (and hence, lesser patient discomfort) as the bolus passes through a constriction formed by the compliant band without the restriction device. This illustrates a potential drawback in the compliant band without a restriction device as the patient might not be aware that he or she has eaten an inappropriately large piece of food.

Line **4310** is a time-pressure curve corresponding to a compliant band with a restriction device (e.g., as shown in FIG. 42). As shown by the line **4310**, the pressure may rise quickly causing the patient to feel pressure for a short period of time as the fluid drains slowly from the band (e.g., the gastric band **4205**) and into the reservoir (e.g., the reservoir **4212**). After a short period of time (which may serve as a reminder to the patient to modify his or her eating pattern and to avoid eating large pieces of food) the band drains enough fluid into the reservoir to allow the bolus to pass through, thereby returning the pressure near equilibrium.

Balloons, reservoirs, access ports and flow control devices having been discussed, attention will now be turned to the gastric band portion of a gastric banding system.

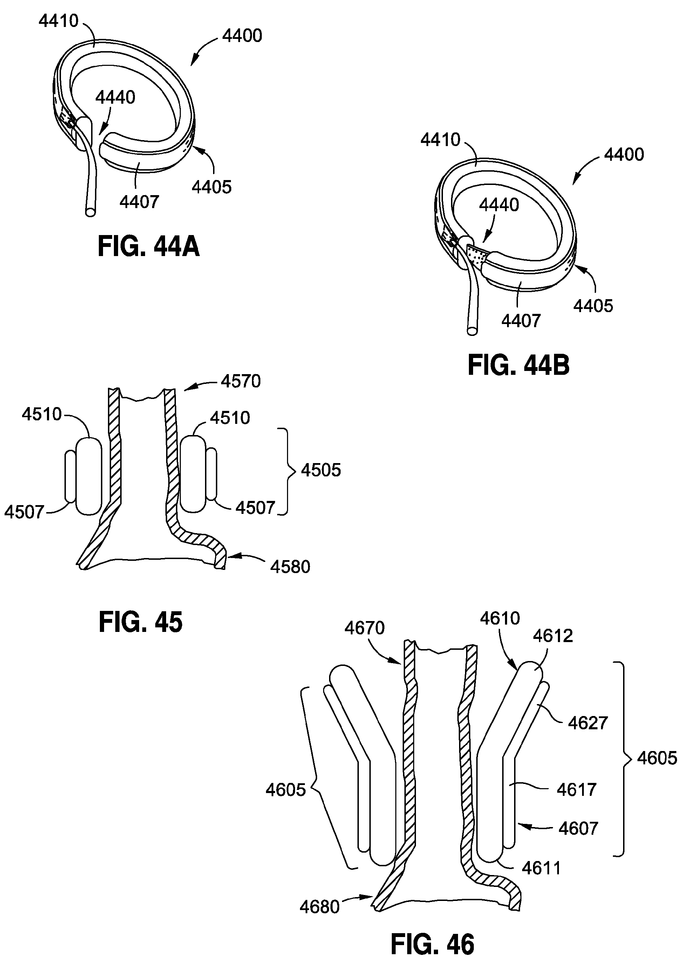

FIG. 44A illustrates an always-open gastric banding system **4400** including a gastric band **4405** having a ring **4407** and an inflatable portion **4410**. Instead of having a flexibly-stiff ring locked in place at an open end (as traditionally utilized in a standard gastric band), the gastric band **4405** does not include a locking portion and replaces the flexibly-stiff ring with a more inflexible ring (e.g., ring **4407**) such as a snap ring, a split ring, retaining ring and the like. The more inflexible ring (e.g., the ring **4407**) may be constructed out of metal and a corresponding spring rate may be adjusted to provide a substantially constant force on the outside of the patient's stomach. The adjustability of the inflatable portion (e.g., the inflatable portions **4410**) may be eliminated. As shown, in this embodiment of FIG. 44, the other components (access port, tubing, etc.) of a standard gastric band system may be removed. In operation, once the gastric banding system **4400** is implanted, the gastric band **4405** may flex open when a large bolus moves through the constriction of the gastric band **4405** on the stomach region. Once the large bolus passes the constriction of the gastric band **4405**, the gastric band **4405** may return to its original orientation. As shown, the gastric banding system **4400** may include a variable width gap **4440** between the opposite ends of the gastric band **4405**. The gap **4440** may serve to provide a more effective response to a patient's anatomy and physiological conditions (e.g., swallowing a large bolus).

FIG. 44B illustrates another form of the gastric banding system **4400** of FIG. 44A. As shown, the gastric banding system **4400** further includes elastic members **4450**, which may be tethered to "close" the variable width gap **4440**. In this embodiment, the elastic members **4450** may be constructed out of a flexible polymer and may function to enhance the ability of the gastric banding system **4400** to remain in place.

In addition to and/or as an alternative to gastric banding systems having an "open" configuration (e.g., the gastric banding system **4400**), a gastric band may also be configured to have portions of various sizes. FIG. 45 illustrates a cross-sectional view of the standard gastric band **4505** having a ring **4507** providing structural support to an inflatable portion **4510.** As shown, the standard gastric band **4505** is placed or fixed about the esophageal-gastric junction between a patient's esophagus **4570** and stomach **4580.**

FIG. 46 illustrates a system with a wider section. As shown in the cross-sectional view of FIG. 46, the improved gastric band **4605** may still include a ring **4607** and an inflatable portion **4610**. However, the design of the ring **4607** may include a standard portion **4617** and a wider portion **4627**, and the inflatable portion **4610** may include a corresponding standard portion **4611** and a corresponding wider portion **4612**. The wider portions **4612** and **4627** may be placed orad (closer or toward the mouth of the patient) as compared to the standard portions **4611** and **4617**. The wider portions **4612** and **4627** may operate to stimulate and restrict the patient's esophageal-gastric junction when the patient is not eating or swallowing small boluses. When the patient swallows medium-sized boluses, the wider portions **4612** and **4627** may channel the medium bolus through the standard portions **4611** and **4617**. And when the patient swallows large boluses, the wider portions **4612** and **4627** may function to relieve the stress on the patient's tissue and assist to prevent formations of pouch dilatations. The wider portions **4612** and **4627**, as shown in FIG. 46, may increase in diameter in the orad direction. Optional suture tabs (not shown) may also be added to the gastric band **4605**, for example, when the additional width of the wider portions **4612** and **4627** render it preferable that a standard gastro-gastric suturing process is not used. The wider portions **4612** and **4627** may be adjustable.

The wider portions **4612** and **4627** may have the same properties as the standard portions **4611** and **4617** (e.g., same materials, durometer, balloon-to-ring width ratio). Additionally, and/or as an alternative, the height of the ring **4607** and/or the height of the inflatable portion **4610** may be adjustable and might not span the entire height of the gastric band **4600** (not shown).

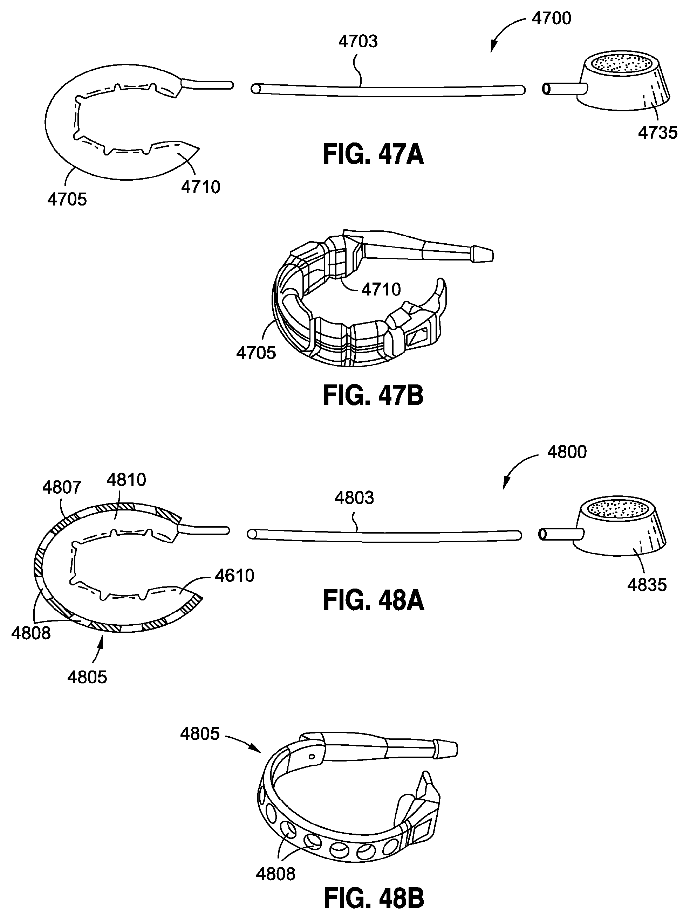

Further configurations of a gastric band system may include alterations to the ring portion. For example, FIG. 47A illustrates a gastric banding system **4700** having a gastric band **4705**, a tube **4703** and an access port **4735**. As shown, the gastric band **4705** may include an inflatable portion **4710** without the presence of a ring. By eliminating the ring, the gastric banding system **4700** may be more compliant. As shown, the gastric banding system **4700** may rely on the inflatable portion **4710** alone for ring structure. FIG. 47B illustrates a perspective view of the gastric band **4705** without the ring structure, and with the other structures of the gastric banding system **4700** omitted.

FIG. 48A illustrates another embodiment of a gastric banding system **4800** having a gastric band **4805**, a tube **4803** and an access port **4835**. As shown, the gastric band **4805** may include inflatable portion **4810** and modified ring **4807** having holed portions **4808**. The addition of the holed portions **4808** may increase the compliance of the gastric banding system **4800.** FIG. 48B illustrates a perspective view of the gastric band **4805** with holed portions **4808**. The inflatable portion **4810** and the other structures of the gastric banding system **4800** have been omitted for clarity.

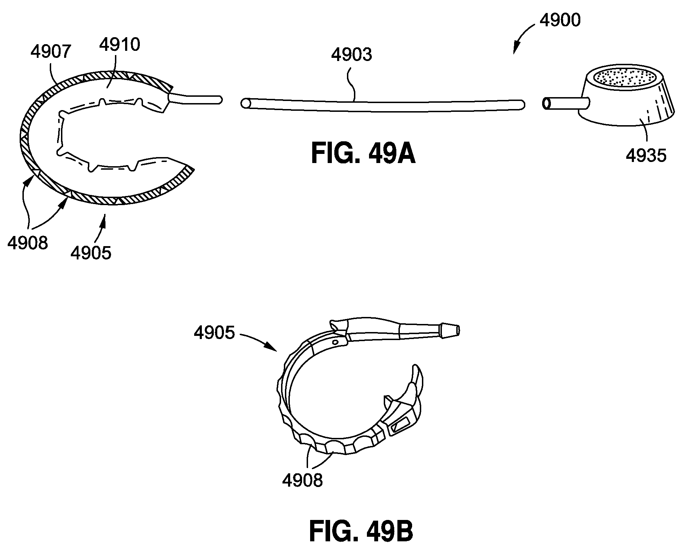

FIG. 49A illustrates another gastric banding system **4900** having a gastric band **4905**, a tube **4903** and an access port **4935.** As shown, the gastric band **4905** may include an inflatable portion **4910** and a modified ring **4907** having cut-out or tapered portions **4908.** The cut-out or tapered portions **4908** may increase the compliance of the gastric banding system **4900.** FIG. 49B illustrates a perspective view of the gastric band **4905** with the cut-out or tapered portions **4908**. The inflatable portion **4910** and the other structures of the gastric banding system **4900** have been omitted for clarity.

The gastric bands **4705, 4805** and **4905** may be more flexible (e.g., by having decreased ring stiffness) than a standard gastric band, thereby resulting in gastric banding systems **4700, 4800** and **4900**, respectively, having muted pressure or force spikes on the tissues (e.g., in the esophageal-gastric junction) in a patient when the patient consumes a large bolus of food. Furthermore, each of the gastric bands **4705, 4805** and **4905** may have different, configurable torsional and ring stiffness characteristics to further create an ergonomically ideal function to serve the patient.

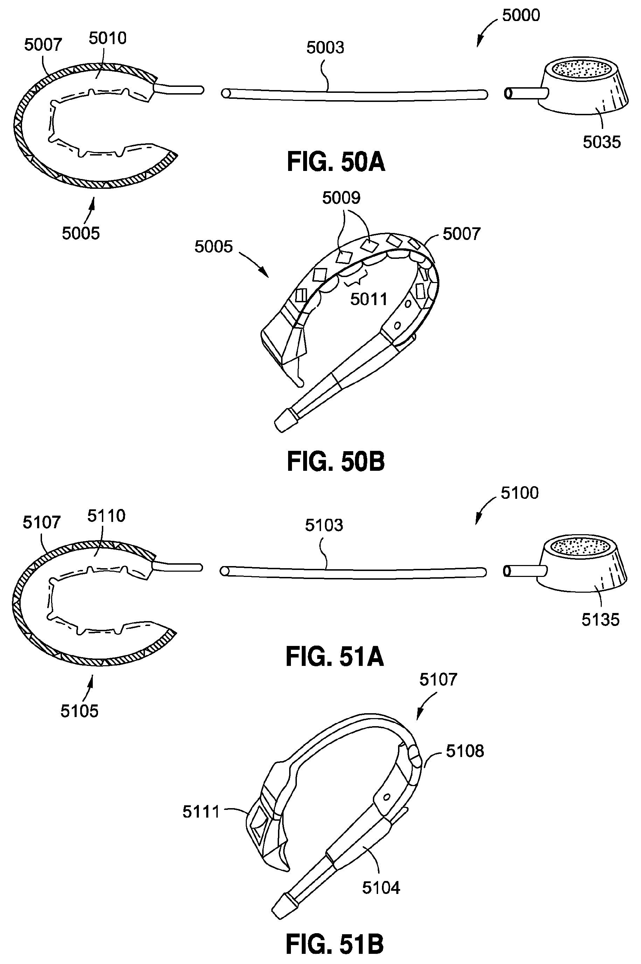

In additional to gastric bands **4705, 4805** and **4905**, a gastric band **5005** of a gastric banding system **5000** as illustrated in FIG. 50A may serve as another alternative providing the desired compliance performance curve. As shown, FIG. 50A of the gastric banding system **5000** includes the gastric band **5005**, a tube **5003** and an access port **5035**. The gastric band **5005** may also include an inflatable portion **5010** and a modified ring **5007** having a modified surface texture/topography. The modified surface texture/topography may increase the compliance of the gastric banding system **5000**.

FIG. 50B illustrates a perspective view of the gastric band **5005** with the modified surface texture/topography of the modified ring **5007**. The inflatable portion **5010** and the other structures of the gastric banding system **5000** have been omitted for clarity. In this example, the modified surface texture/topography may be a combination of a uniform set of diamond-shaped indentions or apertures **5009** along a length of the ring **5007** such that each diamond-shaped indention or aperture **5009** correlates with a different, inwardly-tapered segment-like portion **5011** of the ring **5007**.

FIG. 51A may serve as another alternative providing the desired compliance performance curve. As shown, the gastric banding system **5100** includes a gastric band **5105**, a tube **5103** and an access port **5135**. The gastric band **5105** may also include an inflatable portion **5110** and a modified ring **5107** having a tapered or thin body portion. The tapered body portion may increase the compliance of the gastric banding system **5000** by increasing the flex of the ring **5107**.

FIG. 51B illustrates a perspective view of the gastric band **5105** with the tapered body portion **5108**. The inflatable portion **5110** and the other structures of the gastric banding system **5100** have been omitted for clarity. In this example, the tapered body portion **5108** may be a bar connecting the head **5104** (e.g., buckle) to a tail **5111** (e.g., belt) along a length of the ring **5107.**

FIGS. 50A - 50B and FIGS. 51A - 51B merely illustrate examples of different gastric bands according to the invention that may be utilized to customize the compliance performance curve. However, one skilled in the art will recognize that, while not shown, other embodiments may include additional cuts, breaks, and/or strategic layering using adhered or sliding surfaces to fine tune the compliance curve. In addition and/or alternatively, increasing the length of the ring (e.g., the ring **5007, 5107**) may further tune the compliance curve.

The compliance, stiffness uniformity, or assembly strength of an overall gastric banding system may further be altered by varying the length, surface texture/topography, geography, taper of the end features (e.g., the area closest to the tail or buckle of the gastric band). Also, utilizing various assembly compositions, segments, portions or pieces of stiffer or softer material suspended in the band may adjust the resulting compliance of the overall band.

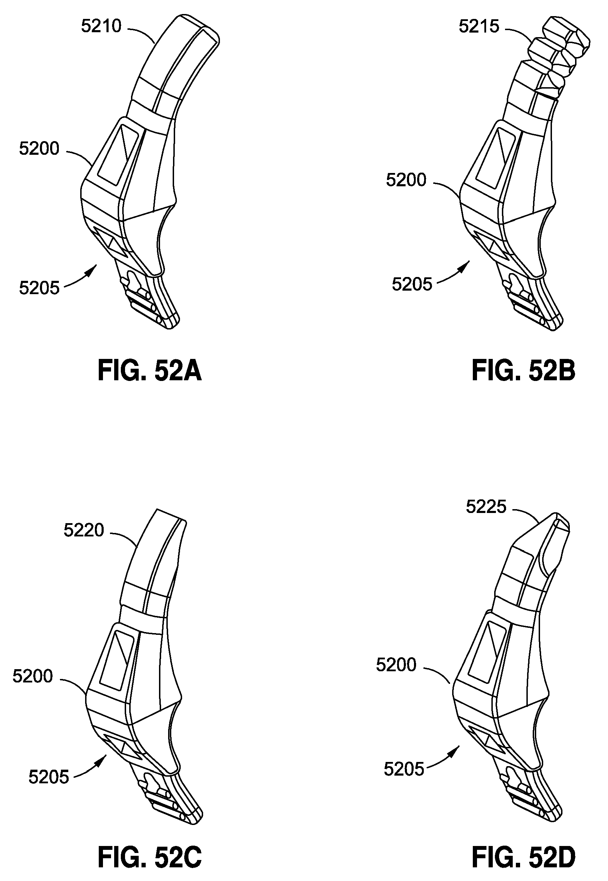

For instance, FIGS. 52A-52D illustrate four examples of tails or buckles of a gastric band with various over-molded interface features. Other portions of the gastric band such as the body of the ring (e.g., body **5108**) and the head (e.g., head or belt **5111**) have been omitted for clarity. However, any of the examples shown in FIGS. 52A-52D may be compatible with any of the gastric bands described herein or known in the art.

FIG. 52A illustrates a buckle or a tail **5200** having an insertion portion **5205** to receive a belt or head (not shown). In addition, the tail **5200** may include an interface **5210** for attaching to the body of the ring (not shown). Here, the interface **5210** is shown to be substantially uniform in shape having four substantially similar smooth faces.

FIG. 52B also illustrates the buckle or tail **5200** having the insertion portion **5205**, but attached to an interface **5215**. Here, the interface **5215** has many cut-out portions on a surface substantially parallel to the insertion portion **5205** while having pyramid-shaped protrusions along a surface substantially orthogonal to the insertion portion **5205**. As compared to FIG. 52A, the more complex surface topography as embodied in the cut-out portions on the interface **5215** of FIG. 52B may increase the compliance of the tail **5200**.

FIG. 52C illustrates the buckle or tail **5200** having the insertion portion **5205**, but attached to an interface **5220**. The interface **5220** may be similar to the interface **5210** of FIG. 52A with one important distinction - namely that the interface **5220** tapers on one side as it extends away from the insertion portion **5205**. The tapering may improve the compliance of the tail **5200**.

FIG. 52D illustrates the buckle or tail **5200** having the insertion portion **5205**, but attached to an interface **5225**. The interface **5225** may be similar to the interface **5220** of FIG. 52C with one important distinction - namely that the interface **5225** tapers on multiple sides as it extends away from the insertion portion **5205**. The additional tapering may further improve the compliance of the tail **5200**.

Such examples serve to illustrate various degrees of compliance. Different patients may benefit from different gastric banding system compliances and these examples of ring variations may serve to fine tune the overall compliance of a gastric banding system to provide patients with a customizable compliance.

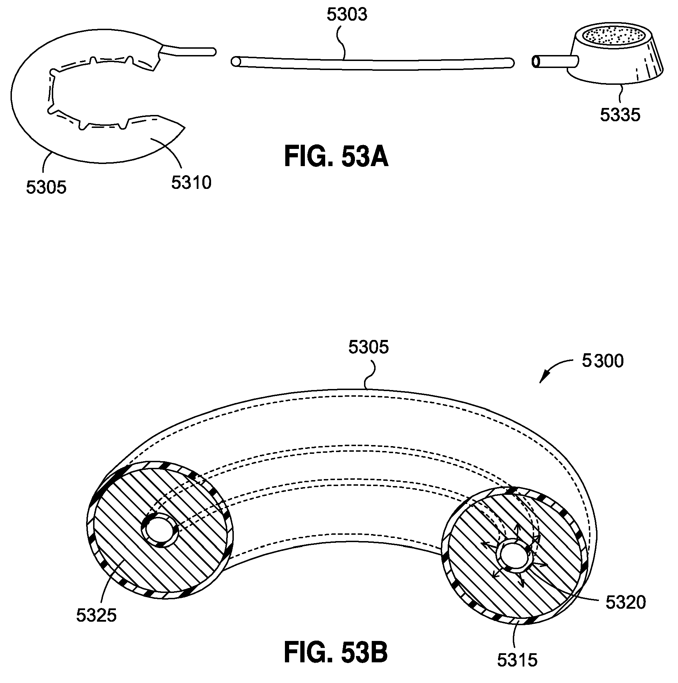

FIG. 53A illustrates a dynamic ring gastric banding system **5300** which may include a gastric band **5305**, a tube **5303** and an access port **5335**. The gastric band **5305** may also include an inflatable portion **5310**. The gastric band **5305** is obstruction-tolerant and may utilize non-fluid transfer means directly integrated into the gastric band itself. More particularly, the inflatable portion **5310** may be filled with a highly compliant, easily compressible or incompressible material medium. For example, the material fill may be incompressible, yet highly compliant and elastic (e.g., a gel) or compressible and non-elastic (e.g., a foam or mesh matrix).

FIG. 53B illustrates a cross-sectional view of the gastric band **5305** of FIG. 53A. As shown, the gastric band **5305** may include an outer shell **5315**, a compliant core tubing **5320** in fluid communication with the rest of the gastric banding system **5300** (as shown in FIG. 53A). The compliant core tubing **5320** may be an inner tubing having a particular length and diameter. The outer shell **5315** may span (or be disposed about) the length of the compliant core tubing **5320** and may have a diameter greater than the diameter of the compliant core tubing **5320**. The compliant core tubing **5320** may be surrounded by a material medium **5325** such as a gel, foam or mesh matrix.

The compliant core tubing **5320** may be a fluid lumen for saline injection and may expand when filled with increased volumes of saline. In one embodiment, when the compliant core tubing **5320** expands with saline, the material medium **5325** may be compressed, causing the gastric band to be less compliant and less tolerant to obstruction. By adjusting the saline or fluid fill, the compliance of the gastric band may be controlled.

By utilizing gel or other fill materials, the compressibility and compliance within the gastric band **5305** may be promoted. For example, where the inflatable portion of the gastric band **5305** is fixed at a maximum volume, the gel provides compressibility within that volume via the material properties of the gel. Accordingly, when a bolus of food passes through the constriction of the gastric band **5305**, the gel may compress itself into the available volume within the gastric band **5305**. The compliant core tubing **5320** which runs through the gel medium and is also contained within the inflatable portion of the gastric band **5305**, acts as a medium by which an incompressible fluid (e.g., saline) when filled in the compliant core tubing **5320**, occupies some of the inflatable volume. By occupying more of the inflatable volume, the compressible fill material has less volume to compress. Furthermore, the compliant core tubing **5320** allows adjustments via saline injection/removal such that when more saline is injected, the more volume within the band is occupied, leaving less volume for the compressible medium to act when a bolus passes through the constriction, thus increasing the sense of restriction.

This allows for dynamic resistance to be built directly into the basic frame and structure of existing gastric band technology. Furthermore, this embodiment is passive and the dynamic resistance may be inherent to the material properties of the design. Also, it provides the benefit of reducing the need for additional chambers, external reservoirs or fluid flow between the chambers and/or reservoirs.

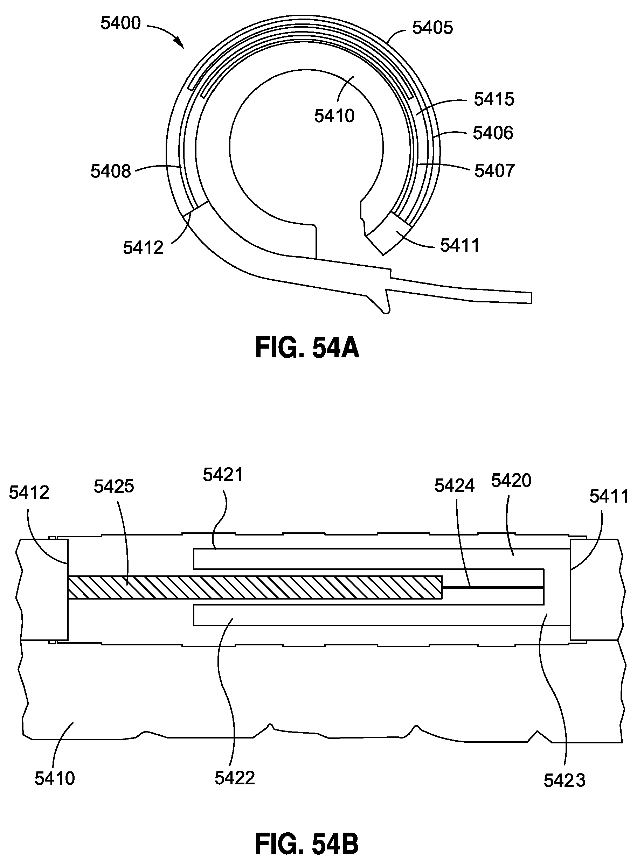

FIG. 54A illustrates a gastric band **5400** which may include a ring **5405** having tubes, rods or strips **5406, 5407** and **5408** constructed out of any of a plurality of materials such as metals, polymers, and the like. These tubes, rods or strips **5406, 5407** and **5408** may be immersed in viscous fluid within a chamber **5415** located on the outside of a fluid reservoir **5410,** which may be in fluid communication with other portions of the gastric banding system. Some other portions of the gastric banding system have been removed for clarity. As shown, the ring **5405** primarily includes the chamber **5415** which has a first and second tube, rod or strip **5406** and **5407** attached to a first end **5411** of the ring **5405,** while the third tube, rod or strip **5408** is attached to the second end **5412** of the ring **5405** and located proximally between the first and second tube, rod or strip **5406** and **5407.** By immersing these structures (e.g., the first, second and third tube, rod or strips **5405, 5406** and **5407**) in viscous fluid, unique physical characteristics such as speed limiters and viscosity effects that manipulate the movement and forcing characteristics of the gastric band **5400** when under load may be obtained. Generally, when a large load is applied, the fluid and the tubes, rods or strips **5406, 5407, 5408** work to strongly resist, and when the load is small, the fluid and the tubes, rods or strips **5406, 5407, 5408** do not provide much resistance. Certain examples of fluids which may be used include but are not limited to thixotropic, viscoelastic and Bingham-type fluids.

FIG. 54B illustrates a close-up view with an alternative structure. Here, between the first end **5411** and the second end **5412** of the ring **5405** exists a chamber **5415** filled with viscous fluid and a first tube, rod or strip **5420** and a second tube, rod or strip **5425**. The first tube, rod or strip **5420** may include a first portion **5421**, a second portion **5422** and a joining portion **5423** which joins the first portion **5421** to the second portion **5422** and also attaches the first tube, rod or strip **5420** to the first end **5411** of the ring. The second tube, rod or strip **5425** may be attached to the second end **5412** of the ring **5405** and may be positioned between the first portion **5421** and the second portion **5422** of the first tube, rod or strip **5420**. The second tube, rod or strip **5425** may also be attached to the joining portion **5423** of the first tube, rod or strip **5420** via a spring, band or string **5424**. In this manner, when a load is applied (e.g., a bolus passing through the constriction formed by the ring **5405**), the first tube, rod or strip **5420** may be pulled away from the second tube, rod or strip **5423** but still remain tethered by the stretched ring, band or string **5424**. Similar to the configuration of FIG. 54A, the structural configuration of FIG. 54B provides a system which acts to strongly resist displacement (temporarily) when a large load is applied, and to easily displace when the load is small.

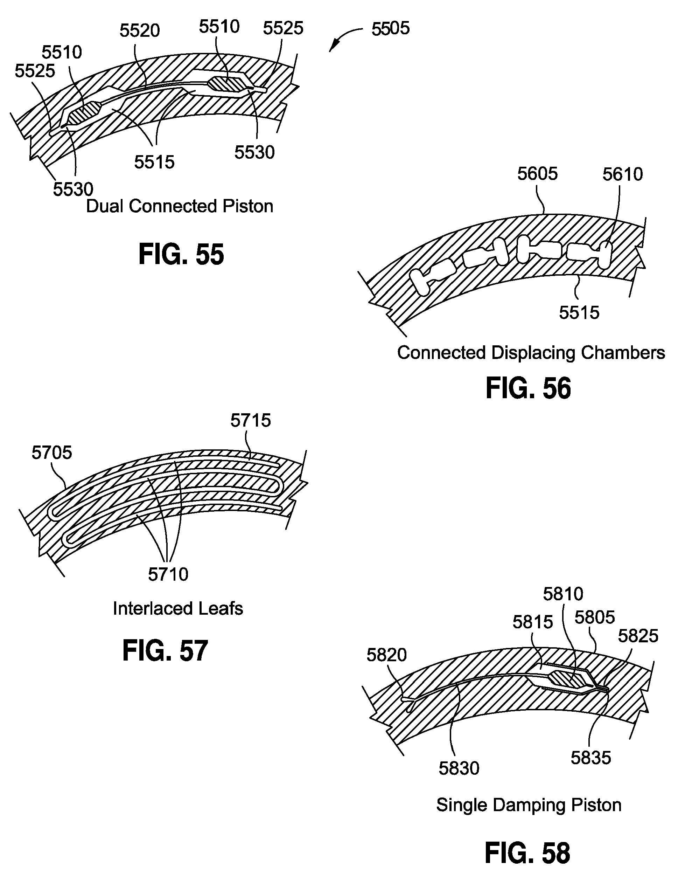

FIGS. 55-58 illustrate additional alternatives of how the ring portion (e.g., ring **5405**) may be modified to strongly resist displacement (temporarily) when a large load is applied, and to easily displace when the load is small. More particularly, a damping mechanism may be employed to resist displacement when a load greater than a predetermined threshold is applied, and to not resist displacement when a load smaller than a predetermined threshold is applied.

For example, as shown in FIG. 55, dual connected pistons **5510** may be utilized to achieve the desired characteristics. The pistons **5510** may be located in their respective orifices **5515**, and immersed with thixotropic, viscoelastic and/or Bingham-type fluids. The pistons **5510** may be tethered to one another by a connecting band **5520**. The pistons **5510** are respectively fixed to an anchor point **5525** via springs **5530**. As shown, the orifices **5515** may be cavities formed within the ring **5505** via, for example, molding. The size of the orifices **5515** (e.g., length) and the distance between the orifices **5515** may define the compliance of the ring **5505**. As a bolus engages the constriction and applies a load to the ring **5505**, the pistons **5510** may move away from one another and migrate from the proximal end of the orifices **5515** to the distal end of the orifices **5515**. However, the pistons **5510** may limit the ring **5505** from further expansion when they press against the distal end of the orifices **5515**. As the bolus passes through the constriction, the pistons **5510** may move back towards one another.

FIG. 56 illustrates a plurality of distinct sets of connected displacing chambers **5610** within a ring **5605**. The chambers **5610** may be filled with thixotropic, viscoelastic and/or Bingham-type fluids. As a bolus engages the constriction and applies a load to the ring **5605**, the fluid characteristics may cause the ring **5605** to strongly resist displacement (temporarily) when a large load is applied, and to easily displace when the load is small.

FIG. 57 illustrates interlaced leaves **5710** immersed within thixotropic, viscoelastic and/or Bingham-type fluids. As a bolus engages the constriction and applies a load to a ring **5705**, the fluid characteristics may cause the ring **5705** to strongly resist displacement (temporarily) when a large load is applied, and to easily displace when the load is small.

FIG. 58 illustrates a single damping piston. The piston **5810** may be tethered to anchor points **5820** and **5825** on opposite sides of an orifice **5815** housing the piston **5820** by connecting bands **5830** and **5835**. The connecting bands **5830** and **5835** may have different (e.g., opposite) tension such that when a load is applied, the band **5830** may displace in one manner (shorten) while the band **5835** displaces in another manner (lengthens) thereby causing the piston **5820** to move from one end of the orifice **5815** to the other end of the orifice **5815**. As shown, the orifice **5815** may be a cavity formed within a ring **5805** via, for example, molding. The size of the orifice **5815** (e.g., length) along with the tension of the connecting bands **5830** and **5835** may define the compliance of the ring **5805**. The orifice **5810** may also be filled with a thixotropic, viscoelastic and/or Bingham-type fluid which may have fluid characteristics causing the ring **5505** to strongly resist displacement (temporarily) when a large load is applied, and to easily displace when the load is small.

While other structural configurations are possible to provide the effects described above, certain embodiments have been disclosed to clarify the concepts.

Other methods for dialing in gastric band performance curves and characteristics include utilizing force threshold mechanisms such as release hooks or magnets that release under a specific increased load. This may allow the gastric band to expand quickly after the limit load is reached. After the load and subsequent expansion passes, the stressed structure of the gastric band brings the load limit device back together in a closed, set configuration for operation under normal loads. This device will then remain closed, providing normal restriction and compliance until a load greater than a predetermined threshold is reached. This concept is intended to maintain the normal operating performance of the gastric band while adding a security feature to temporarily loosen the gastric band under extreme loading conditions to prevent patient injury. By varying the number, size and strength of the magnets as well as the allowable magnet separation when expanded and characteristics of the surrounding structural geometry, the resulting performance curves may be customized.

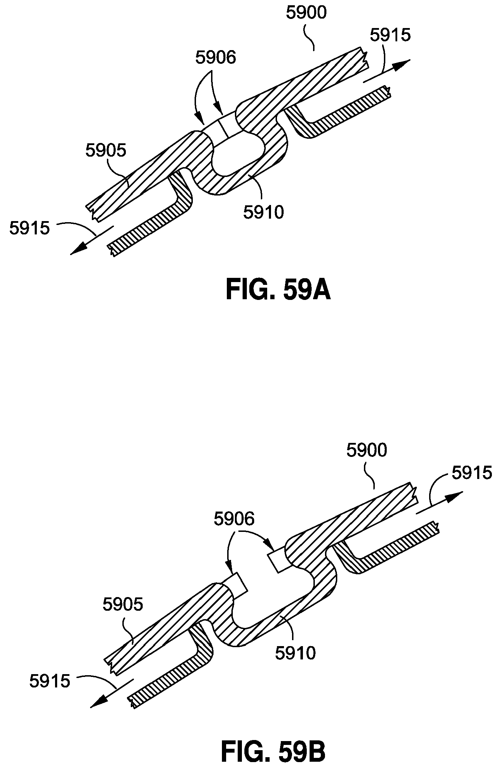

FIGS. 59A and 59B illustrate a load limit device **5906** integrated into a ring **5905** of a gastric band **5900**. FIG. 59A illustrates the load limit device **5906** in a first state (closed). Here, the load limit device **5906** may be a pair of magnets which are attracted to and in contact with one another. The load limit device **5906** may be positioned opposite to the structural member **5910**. When a load (e.g., caused by a bolus passing through the constriction of the gastric band **5900**) is applied in the direction of arrows **5915** great enough to separate the load limit device **5906**, the magnets of the load limit device **5906** may release from one another, thereby expanding the ring **5905** under excess load to allow the bolus to pass through. FIG. 59B illustrates the ring **5905** when the load limit device **5906** is separated under an excess load. Once the bolus passes through, the structural member **5910** (which may be loaded under expansion) may return the load limit device **5906** back together and return the ring **5905** back to the configuration as shown in FIG. 59A. The structural member **5910** may be preloaded in tension or compression while the load limit device **5906** is in the closed position of FIG. 59A to help customize the resulting performance characteristic.

As shown in FIGS. 59A and 59B, the load limit device **5906** includes magnets. However, release hooks or other components which are capable of releasing upon introduction of a load and reengaging after the passage of the load may be utilized in addition or in place of the magnets.

In further embodiments, features may be added to a gastric band in order to limit the maximum expansion that can be obtained under high loads (e.g., a load greater than a predetermined threshold). These features may be configured to work in conjunction with the elastomeric backbone of the gastric band to limit the increased stretch that might occur in certain gastric band designs, thereby allowing for a region of linear elastic expansion with a tunable maximum expansion that prevents over expansion and/or material failure. For example, a stretch limiter may be a conformally flexible but inelastic component that may take the form of a wire, monofilament or strap. Additionally and/or alternatively, the stretch limiter may be a structural, biocompatible material such as titanium wire, polypropylene filament, Teflon, titanium, very high strength and modulus silicone and/or any other appropriate material.

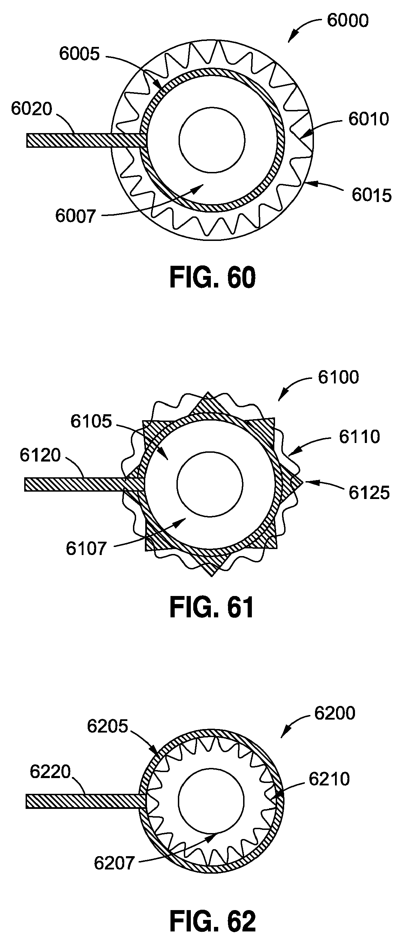

FIG. 60 illustrates an example of features which may limit the maximum expansion of a gastric band **6000**. As shown, the gastric band **6000** may include standard components such as a ring **6005** surrounding the inflatable portion **6007** which is in fluid communication with the rest of the gastric banding system (not shown) via the tubing **6020**. In addition, the gastric band **6000** may include a stretch limiter **6010** and a protective capsule **6015**. The stretch limiter **6010** may be encapsulated in a chamber adjacent to the ring **6005** of the gastric band **6000**, and may be made from material and processes similar to the over-molded reservoir of the typical gastric band. Here, the protective capsule **6015** may be separated from the reservoir **6007** and collapsed/evacuated over the stretch limiter **6010**. With the gastric band **6000** in a relaxed state, the stretch limiter **6010** may be loose with a predetermined amount of slack. As the gastric band **6000** becomes over-expanded, the stretch limiter **6010** may extend outwards and when fully extended, may contact the inner wall of the protective capsule **6015**, and prevent further expansion.

FIG. 61 illustrates another gastric band **6100** having a stretch limiter **6110**. Here, the stretch limiter **6110** may be attached or laced through limiter guides **6125** on the outer side of the elastomeric ring **6105**. In other words, the tip of the limiter guides **6125** may extend beyond the stretch limiter **6110** via holes, cuts or slits on the stretch limiter **6110** which may be formed via molding. As the inflatable portion **6107** expands, the limiter guides **6125** may press and stretch against the stretch limiter **6110**, thereby increasing tension. However, the stretch limiter **6110**, when expanded to a certain predetermined threshold, may prevent further expansion of the inflatable portion **6107**. In this manner, the overall system may function very similarly to the gastric band **6000** of FIG. 60.

FIG. 62 illustrates yet another example of a gastric band **6200** having a stretch limiter **6210**. Here, the stretch limiter **6210** may be disposed inside the inflatable portion **6207** and as fluid volume increases within the inflatable portion **6207**, the stretch limiter **6210** may expand to contact the outer wall of the inflatable portion **6207**. In this example, the stretch limiter **6210** may have a very high modulus elastomer that is flexible enough to be loose and with slack inside the inflatable portion **6207**. When fully extended, the stretch limiter **6210** may prevent further expansion of the inflatable portion **6107**. The stretch limiter **6210** may also be sufficiently wide and round as to not adversely affect the structures pressed against it.

The gastric bands described with respect to FIGS. 60-62 may be as shown (extending about substantially the entire circumference of the gastric band) or may be limited to only a portion designated as an elastic region. The elastic region would then serve as a pressure relief or regulator for the gastric band and the stretch limiters utilized therein would ensure the structural integrity of the elastic region.

Unless otherwise indicated, all numbers expressing quantities of ingredients, volumes of fluids, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, certain references have been made to patents and printed publications throughout this specification.

Specific embodiments disclosed herein may be further limited in the claims using consisting of or and consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. A self-adjusting gastric band system (5000) for the treatment of obesity that adjusts to allow a bolus to pass through a constriction in a patient's stomach, the self-adjusting gastric band system comprising:
a gastric band (5005) having an inner portion, an outer portion, and an inflatable portion (5010), the inner portion configured to be placed around a portion of the patient's stomach to thereby create the constriction in the patient's stomach;
a ring (5007) attached to the outer portion of the gastric band;
an access port (5035) fluidly coupled to the inflatable portion of the gastric band to fill and drain a fluid into or out of the inflatable portion; and
a compliant reservoir fluidly coupled to the inflatable portion and the access port, the compliant reservoir capable of relaxing the constriction formed in the stomach by the gastric band by receiving fluid from the inflatable portion thereby allowing the bolus to pass through the relaxed constriction;
**characterised in that** the ring has a plurality of segmented portions (5011), wherein each segmented portion includes an aperture (5009).

2. The self-adjusting gastric band system of claim 1 wherein the aperture is diamond-shaped.

3. The self-adjusting gastric band system of claim 2 wherein the segmented portions are inwardly-tapered.

4. The self-adjusting gastric band system of clam 1 wherein the ring further comprises:
a belt attached to a first end of the ring; and
a buckle attached to the second end of the ring configured to receive and secure the belt.

5. A self-adjusting gastric band system (5100) for the treatment of obesity that adjusts to allow a bolus of food to pass through a constriction in a patient's stomach, the self-adjusting gastric band system comprising:
a gastric band (5105) having an inflatable portion (5110) and configured to be positioned around a portion of the patient's stomach;
a ring (5107) attached to the inflatable portion and having a belt (5111) attached to a first end of the ring and a buckle (5104) attached to the second end of the ring and configured to receive and secure the belt;
an access port (5135) fluidly coupled to the inflatable portion of the gastric band to fill and drain the inflatable portion; and
a compliant reservoir fluidly coupled to the inflatable portion and the access port, the compliant reservoir for automatically relaxing the constriction formed by the gastric band and allowing the bolus of food to pass through the constriction by receiving fluid from the inflatable portion;
the ring has a tapered band (5108) positioned between the belt and the buckle.

6. The self-adjusting gastric band system of claim 5 wherein the tapered band is thinner than the belt.

7. The self-adjusting gastric band system of claim 5 wherein the tapered band is thinner than the buckle.

## Patentansprüche

1. Selbsteinstellendes Magenbandsystem (5000) für die Behandlung von Fettleibigkeit, das sich einstellt, um einem Bolus zu ermöglichen, durch eine Verengung in einem Patientenmagen zu gelangen, wobei das selbsteinstellende Magenbandsystem aufweist:
ein Magenband (5005), das einen inneren Abschnitt, einen äußeren Abschnitt und einen aufblasbaren Abschnitt (5010) aufweist, wobei der innere Abschnitt eingerichtet ist, um einen Abschnitt des Patientenmagens platziert zu sein und dadurch die Verengung in dem Patientenmagen zu erzeugen;
ein Ring (5007), der an dem äußeren Abschnitt des Magenbands angebracht ist;
ein Zugangsanschluss (5035), der mit dem aufblasbaren Abschnitt des Magenbands in Fluidverbindung steht, um ein Fluid in den aufblasbaren Abschnitt zu füllen oder aus diesem abzulassen; und
ein nachgiebiges Reservoir, das mit dem aufblasbaren Abschnitt und dem Zugangsanschluss in Fluidverbindung steht, wobei das nachgiebige Reservoir imstande ist, die durch das Magenband in dem Magen ausgebildete Verengung durch Empfangen von Fluid von dem aufblasbaren Abschnitt zu entspannen, wodurch dem Bolus ermöglicht wird, durch die entspannte Verengung zu gelangen;
**dadurch gekennzeichnet, dass** der Ring eine Vielzahl segmentierter Abschnitte (5011) aufweist, wobei jeder segmentierte Abschnitt eine Öffnung (5009) aufweist.

2. Selbsteinstellendes Magenbandsystem nach Anspruch 1, bei dem die Öffnung diamantförmig ist.

3. Selbsteinstellendes Magenbandsystem nach Anspruch 2, bei dem sich der segmentierte Abschnitt nach innen verjüngt.

4. Selbsteinstellendes Magenbandsystem nach Anspruch 1, bei dem der Ring ferner aufweist:
einen Gurt, der an einem ersten Ende des Rings angebracht ist; und
eine Schnalle, die an dem zweiten Ende des Rings angebracht ist und eingerichtet ist, den Gurt zu empfangen und zu sichern.

5. Selbsteinstellendes Magenbandsystem (5100) für die Behandlung von Fettleibigkeit, das sich einstellt, um es einem Lebensmittelbolus zu ermöglichen, durch eine Verengung in einen Patientenmagen zu gelangen, wobei das selbsteinstellende Magenbandsystem aufweist:
ein Magenband (5105), das einen aufblasbaren Abschnitt (5110) aufweist und eingerichtet ist, um einen Abschnitt des Patientenmagens positioniert zu sein;
einen Ring (5107), der an dem aufblasbaren Abschnitt angebracht ist und einen Gurt (5111), der an einem ersten Ende des Rings angebracht ist, und eine Schnalle (5104) aufweist, die an dem zweiten Ende des Rings angebracht ist und eingerichtet ist, den Gurt zu empfangen und zu sichern;
einen Zugangsanschluss (5135), der mit dem aufblasbaren Abschnitt des Magenbands in Fluidverbindung steht, um den aufblasbaren Abschnitt zu füllen und zu entleeren; und
ein nachgiebiges Reservoir, das mit dem aufblasbaren Abschnitt und dem Zugangsanschluss in Fluidverbindung steht, wobei das nachgiebige Reservoir für ein automatisches Entspannen der durch das Magenband ausgebildeten Verengung ist und es dem Lebensmittelbolus durch Empfangen von Fluid von dem aufblasbaren Abschnitt ermöglicht, durch die Verengung zu gelangen;
wobei der Ring ein sich verjüngendes Band (5108) aufweist, das zwischen dem Gurt und der Schnalle positioniert ist.

6. Selbsteinstellendes Magenbandsystem nach Anspruch 5, bei dem das sich verjüngende Band dünner ist als der Gurt.

7. Selbsteinstellendes Magenbandsystem nach Anspruch 5, bei dem das sich verjüngende Band dünner ist als die Schnalle.

## Revendications

1. Système d'anneau gastrique à auto-ajustement (5000) pour le traitement de l'obésité, qui s'ajuste pour permettre le passage d'un bolus à travers une constriction dans l'estomac d'un patient, le système d'anneau gastrique à auto-ajustement comprenant :
Un anneau gastrique (5005) ayant une partie intérieure, une partie extérieure et une partie gonflable (5010), la partie intérieure étant conçue pour être placée autour d'une partie de l'estomac du patient pour ainsi créer la constriction dans l'estomac du patient ;
un anneau (5007) fixé à la partie extérieure de l'anneau gastrique ;
un orifice d'accès (5035) couplé par voie fluidique à la partie gonflable de l'anneau gastrique pour remplir d'un fluide la partie gonflable et l'en drainer ; et
un réservoir souple couplé par voie fluidique à la partie gonflable et à l'orifice d'accès, le réservoir souple étant capable de relâcher la constriction formée dans l'estomac par l'anneau gastrique en recevant le fluide de la partie gonflable, permettant ainsi le passage du bolus à travers la constriction relâchée ;
**caractérisé en ce que** l'anneau a une pluralité de parties segmentées (5011), chaque partie segmentée comprenant une ouverture (5009).

2. Système d'anneau gastrique à auto-ajustement selon la revendication 1, dans lequel l'ouverture est en forme de losange.

3. Système d'anneau gastrique à auto-ajustement selon la revendication 2, dans lequel les parties segmentées se rétrécissent vers l'intérieur.

4. Système d'anneau gastrique à auto-ajustement selon la revendication 1, dans lequel l'anneau comprend en outre :
une ceinture fixée à une première extrémité de l'anneau ; et
une boucle fixée à la seconde extrémité de l'anneau conçue pour recevoir et fixer la ceinture.

5. Système d'anneau gastrique à auto-ajustement (5100) pour le traitement de l'obésité, qui s'ajuste pour permettre le passage d'un bolus alimentaire à travers une constriction dans l'estomac d'un patient, le système d'anneau gastrique à auto-ajustement comprenant :
un anneau gastrique (5105) ayant une partie gonflable (5110) et conçu pour être positionné autour d'une partie de l'estomac du patient ;
un anneau (5107) fixé à la partie gonflable et ayant une ceinture (5111) fixée à une première extrémité de l'anneau et une boucle (5104) fixée à la seconde extrémité de l'anneau et conçue pour recevoir et fixer la ceinture ;
un orifice d'accès (5135) couplé par voie fluidique à la partie gonflable de l'anneau gastrique pour remplir et drainer la partie gonflable ; et
un réservoir souple couplé par voie fluidique à la partie gonflable et à l'orifice d'accès, le réservoir souple étant destiné à relâcher automatiquement la constriction formée par l'anneau gastrique et permettre le passage du bolus alimentaire à travers la constriction en recevant le fluide de la partie gonflable ;
l'anneau a une bande effilée (5108) positionnée entre la ceinture et la boucle.

6. Système d'anneau gastrique à auto-ajustement selon la revendication 5, dans lequel la bande effilée est plus mince que la ceinture.

7. Système d'anneau gastrique à auto-ajustement selon la revendication 5, dans lequel la bande effilée est plus mince que la boucle.
